# EUROPEAN PATENT SPECIFICATION

(11) **EP 1 766 047 B1**
(45) Date of publication and mention of the grant of the patent: **23.09.2009**
(21) Application number: 05755214.3
(22) Date of filing: 26.05.2005
(51) Int. Cl.: C12Q 1/26

(54) **HIF PROLYL HYDROXYLASE ACTIVITY ASSAY**
HIF-PROLYLHYDROXYLASE-AKTIVITÄTSTEST
DOSAGE DE L'ACTIVITE PROLYL HYDROXYLASE DE HIF

(30) Priority: 28.05.2004 US 575324 P
(43) Date of publication of application: 28.03.2007
(73) Proprietor: Fibrogen, Inc., San Francisco, CA 94158 (US)
(72) Inventor: BRENNER, Mitchell, C., San Bruno, CA 94066 (US)
(74) Representative: Goodfellow, Hugh Robin
(86) International application number: PCT/US2005/018577
(87) International publication number: WO 2005/118836

(56) References cited:
- WO-A-02/074981
- WO-A-03/049686
- HUANG JIANHE ET AL: "Sequence determinants in hypoxia-inducible factor-1alpha for hydroxylation by the prolyl hydroxylases PHD1, PHD2, and PHD3." JOURNAL OF BIOLOGICAL CHEMISTRY, vol. 277, no. 42, 18 October 2002 (2002-10-18), pages 39792-39800, XP008053316 ISSN: 0021-9258 cited in the application
- OEHME FELIX ET AL: "A nonradioactive 96-well plate assay for the detection of hypoxia-inducible factor prolyl hydroxylase activity." ANALYTICAL BIOCHEMISTRY, [Online] 10 May 2004 (2004-05-10), XP002347276 SCIENCE DIRECT Retrieved from the Internet: URL:http://www.sciencedirect.com/science?_ ob=IssueURL&_tockey=%23TOC%236692%232004%2 3996699998%23504264%23FLA%23&_auth=y&view= c&_acct=C000049880&_version=1&_urlVersion= 0&_userid=987766&md5=c18396c9d17b7310adf51 82aa312a2dc> [retrieved on 2005-09-29] & OEHME FELIX ET AL: "A nonradioactive 96-well plate assay for the detection of hypoxia-inducible factor prolyl hydroxylase activity." ANALYTICAL BIOCHEMISTRY. 1 JUL 2004, vol. 330, no. 1, 1 July 2004 (2004-07-01), pages 74-80, ISSN: 0003-2697
- HIRSILA MAIJA ET AL: "Characterization of the human prolyl 4-hydroxylases that modify the hypoxia-inducible factor." JOURNAL OF BIOLOGICAL CHEMISTRY, vol. 278, no. 33, 15 August 2003 (2003-08-15), pages 30772-30780, XP008053313 ISSN: 0021-9258 cited in the application
- TAYLOR M S: "Characterization and comparative analysis of the EGLN gene family" GENE: AN INTERNATIONAL JOURNAL ON GENES AND GENOMES, ELSEVIER SCIENCE PUBLISHERS, BARKING, GB, vol. 275, no. 1, 5 September 2001 (2001-09-05), pages 125-132, XP004307119 ISSN: 0378-1119 cited in the application

### SEQUENCE LISTING

<110> FIBROGEN, INC.
   Brenner, Mitchell C.
<120> HIF PROLYL HYDROXYLASE ACTIVITY ASSAY
<130> FP0618 PCT
<140> Not yet assigned
   <141> Concurrently herewith
<150> US 60/575,324
   <151> 2004-05-28
<160> 92
<170> PatentIn version 3.2
<210> 1
   <211> 93
   <212> PRT
   <213> Unknown
<220>
   <223> PF03171: 2OG-Fe(II) oxygenase superfamily concensus
<400> 1
   Gln Val Ala Arg Tyr Gly Pro Gly Gly Tyr Tyr Gly Pro His Val Asp
   1               5                   10                  15
   Asn Phe Val Asp Asp Glu Asn Gly Asn Arg Ile Ala Thr Phe Leu Leu
               20                  25                  30
   Tyr Leu Asn Asp Val Glu Glu Gly Gly Glu Leu Val Phe Pro Gly Leu
           35                  40                  45
   Arg Ala Asp Val Cys Ala Thr Val Lys Pro Lys Lys Gly Asp Leu Leu
       50                  55                  60
   Phe Phe Pro Ser Gly Asp Gly Arg Ser Leu His Gly Val Cys Pro Val
   65                  70                  75                  80
   Thr Arg Gly Ser Arg Trp Ala Ile Thr Gly Trp Phe Arg
                   85                  90
<210> 2
   <211> 111
   <212> PRT
   <213> Unknown
<220>
   <223> CDD13071: EGL-Nine (EGLN) protein conserved domain
<400> 2
   Met Val Ala Cys Tyr Pro Gly Asn Gly Thr Gly Tyr Val Arg His Val
   1 5 10 15
   Asp Asn Pro His Gly Asp Gly Arg Cys Ile Thr Cys Ile Tyr Tyr Leu
   20 25 30
   Asn Gln Asn Trp Asp Val Lys Val His Gly Gly Ile Leu Arg Ile Phe
   35 40 45
   Pro Glu Gly Ser Thr Thr Phe Ala Asp Ile Glu Pro Lys Phe Asp Arg
   50 55 60
   Leu Leu Phe Phe Trp Ser Asp Arg Arg Asn Pro His Glu Val Gln Pro
   65 70 75 80
   Ala Tyr Ala Thr Arg Tyr Ala Ile Thr Val Trp Tyr Phe Asp Ala Lys
   85 90 95
   Glu Arg Ala Glu Ala Lys Gly Lys Gly Lys Leu Cys Pro Ala Pro
   100 105 110
<210> 3
   <211> 104
   <212> PRT
   <213> Unknown
<220>
   <223> HIF prolyl 4-hydroxylase concensus
<220>
   <221> MISC_FEATURE
   <222> (10)..(10)
   <223> T or L
<220>
   <221> MISC_FEATURE
   <222> (20) .. (20)
   <223> V or N
<220>
   <221> MISC_FEATURE
   <222> (26)..(26)
   <223> V or I
<220>
   <221> MISC_FEATURE
   <222> (34)..(34)
   <223> K, Q, or E
<220>
   <221> MISC_FEATURE
   <222> (35) ..(35)
   <223> D or N
<220>
   <221> MISC_FEATURE
   <222> (38)..(38)
   <223> V, A, or M
<220>
   <221> MISC_FEATURE
   <222> (40)..(40)
   <223> V, L, or T
<220>
   <221> MISC_FEATURE
   <222> (41)..(41)
   <223> S, H, or D
<220>
   <221> MISC_FEATURE
   <222> (46)..(46)
   <223> R or Q
<220>
   <221> MISC_FEATURE
   <222> (52)..(52)
   <223> K, R, or S
<220>
   <221> MISC_FEATURE
   <222> (53)..(53)
   <223> A, P, S, or M
<220>
   <221> MISC_FEATURE
   <222> (54)..(54)
   <223> Q, V, F, or T
<220>
   <221> MISC_FEATURE
   <222> (55)..(55)
   <223> F, V, I, or P
<220>
   <221> MISC_FEATURE
   <222> (57)..(57)
   <223> D or N
<220>
   <221> MISC_FEATURE
   <222> (61)..(61)
   <223> K, L, I, or R
<220>
   <221> MISC_FEATURE
   <222> (79)..(79)
   <223> Q, K, or M
<220>
   <221> MISC_FEATURE
   <222> (81)..(81)
   <223> A, S, or V
<220>
   <221> MISC_FEATURE
   <222> (88).. (88)
   <223> I or M
<220>
   <221> MISC_FEATURE
   <222> (96)..(96)
   <223> D, K, E, or S
<220>
   <221> MISC_FEATURE
   <222> (100)..(100)
   <223> R, E, A, or K
<220>
   <221> MISC_FEATURE
   <222> (103)..(103)
   <223> V, D, K, or A
<400> 3
   Met Val Ala Cys Tyr Pro Gly Asn Gly Xaa Gly Tyr Val Arg His Val
   1               5                   10                  15
   Asp Asn Pro Xaa Gly Asp Gly Arg Cys Xaa Thr Cys Ile Tyr Tyr Leu
               20                  25                  30
   Asn Xaa Xaa Trp Asp Xaa Lys Xaa Xaa Gly Gly Ile Leu Xaa Ile Phe
           35                  40                  45
   Pro Glu Gly Xaa Xaa Xaa Xaa Ala Xaa Ile Glu Pro Xaa Phe Asp Arg
       50                  55                  60
   Leu Leu Phe Phe Trp Ser Asp Arg Arg Asn Pro His Glu Val Xaa Pro
   65                  70                  75                  80
   Xaa Tyr Ala Thr Arg Tyr Ala Xaa Thr Val Trp Tyr Phe Asp Ala Xaa
                   85                  90                  95
   Glu Arg Ala Xaa Ala Lys Xaa Lys
               100
<210> 4
   <211> 20
   <212> PRT
   <213> Homo sapiens
<400> 4
   Asp Leu Asp Leu Glu Met Leu Ala Pro Tyr Ile Pro Met Asp Asp Asp
   1 5 10 15
   Phe Gln Leu Arg
   20
<210> 5
   <211> 21
   <212> PRT
   <213> Homo sapiens
<400> 5
   Glu Leu Asp Leu Glu Thr Leu Ala Pro Tyr Ile Pro Met Asp Gly Glu
   1 5 10 15
   Asp Phe Gln Leu Ser
   20
<210> 6
   <211> 20
   <212> PRT
   <213> Homo sapiens
<400> 6
   Ala Leu Asp Leu Glu Met Leu Ala Pro Tyr Ile Ser Met Asp Asp Asp
   1 5 10 15
   Phe Gln Leu Asn
   20
<210> 7
   <211> 20
   <212> PRT
   <213> Artificial sequence
<220>
   <223> DTD20 synthetic peptide
<400> 7
   Asp Thr Asp Leu Asp Leu Glu Met Leu Ala Pro Tyr Ile Pro Met Asp
   1 5 10 15
   Asp Asp Phe Gln
   20
<210> 8
   <211> 20
   <212> PRT
   <213> Artificial sequence
<220>
   <223> Synthetic peptide
<400> 8
   Asp Thr Asp Leu Asp Trp Glu Ala Leu Ala Pro Tyr Ile Pro Ala Asp
   1 5 10 15
   Asp Asp Phe Gln
   20
<210> 9
   <211> 20
   <212> PRT
   <213> Artificial sequence
<220>
   <223> Synthetic peptide
<400> 9
   Asp Thr Asp Leu Asp Leu Glu Ala Val Ala Pro Tyr Ile Pro Ala Asp
   1 5 10 15
   Asp Asp Phe Gln
   20
<210> 10
   <211> 20
   <212> PRT
   <213> Artificial sequence
<220>
   <223> Synthetic peptide
<400> 10
   Asp Thr Asp Leu Asp Tyr Glu Ala Leu Ala Pro Tyr Ile Pro Ala Asp
   1 5 10 15
   Asp Asp Phe Gln
   20
<210> 11
   <211> 20
   <212> PRT
   <213> Artificial sequence
<220>
   <223> Synthetic peptide
<400> 11
   Asp Thr Asp Leu Asp Leu Glu Ala Leu Ala Pro Tyr Ile Pro Ala Asp
   1 5 10 15
   Asp Asp Phe Gln
   20
<210> 12
   <211> 20
   <212> PRT
   <213> Artificial sequence
<220>
   <223> Synthetic peptide
<400> 12
   Asp Thr Asp Leu Asp Leu Glu Ala Ile Ala Pro Tyr Ile Pro Ala Asp
   1 5 10 15
   Asp Asp Phe Gln
   20
<210> 13
   <211> 20
   <212> PRT
   <213> Artificial sequence
<220>
   <223> Synthetic peptide
<400> 13
   Asp Thr Asp Leu Asp Met Glu Ala Leu Ala Pro Tyr Ile Pro Ala Asp
   1 5 10 15
   Asp Asp Phe Gln
   20
<210> 14
   <211> 20
   <212> PRT
   <213> Artificial sequence
<220>
   <223> Synthetic peptide
<400> 14
   Asp Thr Asp Leu Asp Leu Glu Ala Phe Ala Pro Tyr Ile Pro Ala Asp
   1 5 10 15
   Asp Asp Phe Gln
   20
<210> 15
   <211> 20
   <212> PRT
   <213> Artificial sequence
<220>
   <223> Synthetic peptide
<400> 15
   Asp Thr Asp Leu Asp Leu Glu Ala Arg Ala Pro Tyr Ile Pro Ala Asp
   1 5 10 15
   Asp Asp Phe Gln
   20
<210> 16
   <211> 20
   <212> PRT
   <213> Artificial sequence
<220>
   <223> Synthetic peptide
<400> 16
   Asp Thr Asp Leu Asp Leu Glu Ala Tyr Ala Pro Tyr Ile Pro Ala Asp
   1               5                   10                  15
   Asp Asp Phe Gln
               20
<210> 17
   <211> 20
   <212> PRT
   <213> Artificial sequence
<220>
   <223> Synthetic peptide
<400> 17
   Asp Thr Asp Leu Asp Ile Glu Ala Ile Ala Pro Tyr Ile Pro Ala Asp
   1               5                   10                  15
   Asp Asp Phe Gln
               20
<210> 18
   <211> 20
   <212> PRT
   <213> Artificial sequence
<220>
   <223> Synthetic peptide
<400> 18
   Asp Thr Asp Leu Asp Leu Glu Ala Thr Ala Pro Tyr Ile Pro Ala Asp
   1               5                   10                  15
   Asp Asp Phe Gln
               20
<210> 19
   <211> 20
   <212> PRT
   <213> Artificial sequence
<220>
   <223> synthetic peptide
<400> 19
   Asp Thr Asp Leu Asp Phe Glu Ala Leu Ala Pro Tyr Ile Pro Ala Asp
   1 5 10 15
   Asp Asp Phe Gln
   20
<210> 20
   <211> 20
   <212> PRT
   <213> Artificial sequence
<220>
   <223> Synthetic peptide
<400> 20
   Asp Thr Asp Leu Asp Leu Glu Ala Met Ala Pro Tyr Ile Pro Ala Asp
   1 5 10 15
   Asp Asp Phe Gln
   20
<210> 21
   <211> 20
   <212> PRT
   <213> Artificial sequence
<220>
   <223> Synthetic peptide
<400> 21
   Asp Thr Asp Leu Asp Leu Glu Ala Lys Ala Pro Tyr Ile Pro Ala Asp
   1 5 10 15
   Asp Asp Phe Gln
   20
<210> 22
   <211> 20
   <212> PRT
   <213> Artificial sequence
<220>
   <223> Synthetic peptide
<400> 22
   Asp Thr Asp Leu Asp Asp Glu Ala Leu Ala Pro Tyr Ile Pro Ala Asp
   1 5 10 15
   Asp Asp Phe Gln
   20
<210> 23
   <211> 20
   <212> PRT
   <213> Artificial sequence
<220>
   <223> Synthetic peptide
<400> 23
   Asp Thr Asp Leu Asp Ala Glu Ala Leu Ala Pro Tyr Ile Pro Ala Asp
   1 5 10 15
   Asp Asp Phe Gln
   20
<210> 24
   <211> 20
   <212> PRT
   <213> Artificial sequence
<220>
   <223> Synthetic peptide
<400> 24
   Asp Thr Asp Leu Asp Ile Glu Ala Leu Ala Pro Tyr Ile Pro Ala Asp
   1 5 10 15
   Asp Asp Phe Gln
   20
<210> 25
   <211> 20
   <212> PRT
   <213> Artificial sequence
<220>
   <223> Synthetic peptide
<400> 25
   Asp Thr Asp Leu Asp Leu Glu Ala Trp Ala Pro Tyr Ile Pro Ala Asp
   1 5 10 15
   Asp Asp Phe Gln
   20
<210> 26
   <211> 20
   <212> PRT
   <213> Artificial sequence
<220>
   <223> Synthetic peptide
<400> 26
   Asp Thr Asp Leu Asp Glu Glu Ala Leu Ala Pro Tyr Ile Pro Ala Asp
   1 5 10 15
   Asp Asp Phe Gln
   20
<210> 27
   <211> 20
   <212> PRT
   <213> Artificial sequence
<220>
   <223> Synthetic peptide
<400> 27
   Asp Thr Asp Leu Asp Leu Glu Ala Asn Ala Pro Tyr Ile Pro Ala Asp
   1 5 10 15
   Asp Asp Phe Gln
   20
<210> 28
   <211> 20
   <212> PRT
   <213> Artificial sequence
<220>
   <223> Synthetic peptide
<400> 28
   Asp Thr Asp Leu Asp Val Glu Ala Ile Ala Pro Tyr Ile Pro Ala Asp
   1 5 10 15
   Asp Asp Phe Gln
   20
<210> 29
   <211> 20
   <212> PRT
   <213> Artificial sequence
<220>
   <223> Synthetic peptide
<400> 29
   Asp Thr Asp Leu Asp Cys Glu Ala Leu Ala Pro Tyr Ile Pro Ala Asp
   1 5 10 15
   Asp Asp Phe Gln
   20
<210> 30
   <211> 20
   <212> PRT
   <213> Artificial sequence
<220>
   <223> Synthetic peptide
<400> 30
   Asp Thr Asp Leu Asp Leu Glu Ala Cys Ala Pro Tyr Ile Pro Ala Asp
   1 5 10 15
   Asp Asp Phe Gln
   20
<210> 31
   <211> 20
   <212> PRT
   <213> Artificial sequence
<220>
   <223> Synthetic peptide
<400> 31
   Asp Thr Asp Leu Asp Pro Glu Ala Leu Ala Pro Tyr Ile Pro Ala Asp
   1 5 10 15
   Asp Asp Phe Gln
   20
<210> 32
   <211> 20
   <212> PRT
   <213> Artificial sequence
<220>
   <223> Synthetic peptide
<400> 32
   Asp Thr Asp Leu Asp Gln Glu Ala Leu Ala Pro Tyr Ile Pro Ala Asp
   1 5 10 15
   Asp Asp Phe Gln
   20
<210> 33
   <211> 20
   <212> PRT
   <213> Artificial sequence
<220>
   <223> Synthetic peptide
<400> 33
   Asp Thr Asp Leu Asp Gly Glu Ala Leu Ala Pro Tyr Ile Pro Ala Asp
   1 5 10 15
   Asp Asp Phe Gln
   20
<210> 34
   <211> 20
   <212> PRT
   <213> Artificial sequence
<220>
   <223> Synthetic peptide
<400> 34
   Asp Thr Asp Leu Asp Leu Glu Ala Ala Ala Pro Tyr Ile Pro Ala Asp
   1               5                   10                  15
   Asp Asp Phe Gln
               20
<210> 35
   <211> 20
   <212> PRT
   <213> Artificial sequence
<220>
   <223> Synthetic peptide
<400> 35
   Asp Thr Asp Leu Asp Val Glu Ala Tyr Ala Pro Tyr Ile Pro Ala Asp
   1               5                   10                  15
   Asp Asp Phe Gln
               20
<210> 36
   <211> 20
   <212> PRT
   <213> Artificial sequence
<220>
   <223> Synthetic peptide
<400> 36
   Asp Thr Asp Leu Asp Ser Glu Ala Leu Ala Pro Tyr Ile Pro Ala Asp
   1               5                   10                  15
   Asp Asp Phe Gln
               20
<210> 37
   <211> 20
   <212> PRT
   <213> Artificial sequence
<220>
   <223> Synthetic peptide
<400> 37
   Asp Thr Asp Leu Asp Val Glu Ala Phe Ala Pro Tyr Ile Pro Ala Asp
   1 5 10 15
   Asp Asp Phe Gln
   20
<210> 38
   <211> 20
   <212> PRT
   <213> Artificial sequence
<220>
   <223> Synthetic peptide
<400> 38
   Asp Thr Asp Leu Asp Ala Glu Ala Val Ala Pro Tyr Ile Pro Ala Asp
   1 5 10 15
   Asp Asp Phe Gln
   20
<210> 39
   <211> 20
   <212> PRT
   <213> Artificial sequence
<220>
   <223> Synthetic peptide
<400> 39
   Asp Thr Asp Leu Asp Leu Glu Ala His Ala Pro Tyr Ile Pro Ala Asp
   1 5 10 15
   Asp Asp Phe Gln
   20
<210> 40
   <211> 20
   <212> PRT
   <213> Artificial sequence
<220>
   <223> Synthetic peptide
<400> 40
   Asp Thr Asp Leu Asp Ile Glu Ala Asn Ala Pro Tyr Ile Pro Ala Asp
   1 5 10 15
   Asp Asp Phe Gln
   20
<210> 41
   <211> 20
   <212> PRT
   <213> Artificial sequence
<220>
   <223> Synthetic peptide
<400> 41
   Asp Thr Asp Leu Asp Val Glu Ala Leu Ala Pro Tyr Ile Pro Ala Asp
   1 5 10 15
   Asp Asp Phe Gln
   20
<210> 42
   <211> 20
   <212> PRT
   <213> Artificial sequence
<220>
   <223> Synthetic peptide
<400> 42
   Asp Thr Asp Leu Asp Ala Glu Ala Ile Ala Pro Tyr Ile Pro Ala Asp
   1 5 10 15
   Asp Asp Phe Gln
   20
<210> 43
   <211> 20
   <212> PRT
   <213> Artificial sequence
<220>
   <223> Synthetic peptide
<400> 43
   Asp Thr Asp Leu Asp Leu Glu Ala Ser Ala Pro Tyr Ile Pro Ala Asp
   1 5 10 15
   Asp Asp Phe Gln
   20
<210> 44
   <211> 20
   <212> PRT
   <213> Artificial sequence
<220>
   <223> Synthetic peptide
<400> 44
   Asp Thr Asp Leu Asp Asn Glu Ala Leu Ala Pro Tyr Ile Pro Ala Asp
   1 5 10 15
   Asp Asp Phe Gln
   20
<210> 45
   <211> 20
   <212> PRT
   <213> Artificial sequence
<220>
   <223> Synthetic peptide
<400> 45
   Asp Thr Asp Leu Asp Thr Glu Ala Leu Ala Pro Tyr Ile Pro Ala Asp
   1 5 10 15
   Asp Asp Phe Gln
   20
<210> 46
   <211> 20
   <212> PRT
   <213> Artificial sequence
<220>
   <223> Synthetic peptide
<400> 46
   Asp Thr Asp Leu Asp Thr Glu Ala Tyr Ala Pro Tyr Ile Pro Ala Asp
   1               5                   10                  15
   Asp Asp Phe Gln
               20
<210> 47
   <211> 20
   <212> PRT
   <213> Artificial sequence
<220>
   <223> Synthetic peptide
<400> 47
   Asp Thr Asp Leu Asp Ile Glu Ala Val Ala Pro Tyr Ile Pro Ala Asp
   1               5                   10                  15
   Asp Asp Phe Gln
               20
<210> 48
   <211> 20
   <212> PRT
   <213> Artificial sequence
<220>
   <223> Synthetic peptide
<400> 48
   Asp Thr Asp Leu Asp Thr Glu Ala Ile Ala Pro Tyr Ile Pro Ala Asp
   1               5                   10                  15
   Asp Asp Phe Gln
               20
<210> 49
   <211> 20
   <212> PRT
   <213> Artificial sequence
<220>
   <223> Synthetic peptide
<400> 49
   Asp Thr Asp Leu Asp Leu Glu Ala Gln Ala Pro Tyr Ile Pro Ala Asp
   1 5 10 15
   Asp Asp Phe Gln
   20
<210> 50
   <211> 20
   <212> PRT
   <213> Artificial sequence
<220>
   <223> Synthetic peptide
<400> 50
   Asp Thr Asp Leu Asp His Glu Ala Leu Ala Pro Tyr Ile Pro Ala Asp
   1 5 10 15
   Asp Asp Phe Gln
   20
<210> 51
   <211> 20
   <212> PRT
   <213> Artificial sequence
<220>
   <223> Synthetic peptide
<400> 51
   Asp Thr Asp Leu Asp Val Glu Ala Val Ala Pro Tyr Ile Pro Ala Asp
   1 5 10 15
   Asp Asp Phe Gln
   20
<210> 52
   <211> 20
   <212> PRT
   <213> Artificial sequence
<220>
   <223> Synthetic peptide
<400> 52
   Asp Thr Asp Leu Asp Thr Glu Ala Phe Ala Pro Tyr Ile Pro Ala Asp
   1 5 10 15
   Asp Asp Phe Gln
   20
<210> 53
   <211> 20
   <212> PRT
   <213> Artificial sequence
<220>
   <223> Synthetic peptide
<400> 53
   Asp Thr Asp Leu Asp Thr Glu Ala Thr Ala Pro Tyr Ile Pro Ala Asp
   1 5 10 15
   Asp Asp Phe Gln
   20
<210> 54
   <211> 20
   <212> PRT
   <213> Artificial sequence
<220>
   <223> Synthetic peptide
<400> 54
   Asp Thr Asp Leu Asp Ala Glu Ala Thr Ala Pro Tyr Ile Pro Ala Asp
   1 5 10 15
   Asp Asp Phe Gln
   20
<210> 55
   <211> 20
   <212> PRT
   <213> Artificial sequence
<220>
   <223> Synthetic peptide
<400> 55
   Asp Thr Asp Leu Asp Val Glu Ala Thr Ala Pro Tyr Ile Pro Ala Asp
   1 5 10 15
   Asp Asp Phe Gln
   20
<210> 56
   <211> 20
   <212> PRT
   <213> Artificial sequence
<220>
   <223> Synthetic peptide
<400> 56
   Asp Thr Asp Leu Asp Arg Glu Ala Leu Ala Pro Tyr Ile Pro Ala Asp
   1 5 10 15
   Asp Asp Phe Gln
   20
<210> 57
   <211> 20
   <212> PRT
   <213> Artificial sequence
<220>
   <223> Synthetic peptide
<400> 57
   Asp Thr Asp Leu Asp Thr Glu Ala Val Ala Pro Tyr Ile Pro Ala Asp
   1 5 10 15
   Asp Asp Phe Gln
   20
<210> 58
   <211> 20
   <212> PRT
   <213> Artificial sequence
<220>
   <223> Synthetic peptide
<400> 58
   Asp Thr Asp Leu Asp Ala Glu Ala Ala Ala Pro Tyr Ile Pro Ala Asp
   1 5 10 15
   Asp Asp Phe Gln
   20
<210> 59
   <211> 20
   <212> PRT
   <213> Artificial sequence
<220>
   <223> Synthetic peptide
<400> 59
   Asp Thr Asp Leu Asp Leu Glu Ala Gly Ala Pro Tyr Ile Pro Ala Asp
   1 5 10 15
   Asp Asp Phe Gln
   20
<210> 60
   <211> 20
   <212> PRT
   <213> Artificial sequence
<220>
   <223> Synthetic peptide
<400> 60
   Asp Thr Asp Leu Asp Ala Glu Ala Ser Ala Pro Tyr Ile Pro Ala Asp
   1 5 10 15
   Asp Asp Phe Gln
   20
<210> 61
   <211> 20
   <212> PRT
   <213> Artificial sequence
<220>
   <223> Synthetic peptide
<400> 61
   Asp Thr Asp Leu Asp Leu Glu Ala Glu Ala Pro Tyr Ile Pro Ala Asp
   1               5                   10                  15
   Asp Asp Phe Gln
               20
<210> 62
   <211> 20
   <212> PRT
   <213> Artificial sequence
<220>
   <223> Synthetic peptide
<220>
   <221> MISC_FEATURE
   <222> (11)..(11)
   <223> 3,4-dehydroproline
<400> 62
   Asp Thr Asp Leu Asp Leu Glu Ala Leu Ala Xaa Tyr Ile Pro Ala Asp
   1               5                   10                  15
   Asp Asp Phe Gln
               20
<210> 63
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 63
   Asp Thr Asp Leu Asp Leu Glu Ala Leu Ala Xaa Tyr Ile Pro Ala Asp
   1               5                   10                  15
   Asp Asp Phe Gln
               20
<210> 64
   <211> 20
   <212> PRT
   <213> Artificial sequence
<220>
   <223> Synthetic peptide
<220>
   <221> MISC_FEATURE
   <222> (11).. (11)
   <223> Azetidine-2-carboxylic acid
<400> 64
   Asp Thr Asp Leu Asp Val Glu Ala Tyr Ala Xaa Tyr Ile Pro Ala Asp
   1               5                   10                  15
   Asp Asp Phe Gln
               20
<210> 65
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 65
   Asp Thr Asp Leu Asp Thr Glu Ala Tyr Ala Xaa Tyr Ile Pro Ala Asp
   1               5                   10                  15
   Asp Asp Phe Gln
               20
<210> 66
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 66
   Asp Thr Asp Leu Asp Val Glu Ala Phe Ala Xaa Tyr Ile Pro Ala Asp
   1 5 10 15
   Asp Asp Phe Gln
   20
<210> 67
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 67
   Asp Thr Asp Leu Asp Ile Glu Ala Ile Ala Xaa Tyr Ile Pro Ala Asp
   1 5 10 15
   Asp Asp Phe Gln
   20
<210> 68
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 68
   Asp Thr Asp Leu Asp Thr Glu Ala Phe Ala Xaa Tyr Ile Pro Ala Asp
   1 5 10 15
   Asp Asp Phe Gln
   20
<210> 69
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 69
   Asp Thr Asp Leu Asp Thr Glu Ala Leu Ala Xaa Tyr Ile Pro Ala Asp
   1               5                   10                  15
   Asp Asp Phe Gln
               20
<210> 70
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 70
   Asp Thr Asp Leu Asp Ala Glu Ala Ala Ala Xaa Tyr Ile Pro Ala Asp
   1               5                   10                  15
   Asp Asp Phe Gln
               20
<210> 71
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 71
   Asp Thr Asp Leu Asp Ala Glu Ala Val Ala Xaa Tyr Ile Pro Ala Asp
   1 5 10 15
   Asp Asp Phe Gln
   20
<210> 72
   <211> 20
   <212> PRT
   <213> Artificial sequence
<220>
   <223> Synthetic peptide
<220>
   <221> MISC_FEATURE
   <222> (11)..(11)
   <223> b-thioproline
<400> 72
   Asp Thr Asp Leu Asp Leu Glu Ala Leu Ala Xaa Tyr Ile Pro Ala Asp
   1 5 10 15
   Asp Asp Phe Gln
   20
<210> 73
   <211> 20
   <212> PRT
   <213> Artificial sequence
<220>
   <223> Synthetic peptide
<220>
   <221> MISC_FEATURE
   <222> (11) .. (11)
   <223> Azetidine-2-carboxylic acid
<400> 73
   Asp Thr Asp Leu Asp Val Glu Ala Ile Ala Xaa Tyr Ile Pro Ala Asp
   1 5 10 15
   Asp Asp Phe Gln
   20
<210> 74
   <211> 20
   <212> PRT
   <213> Artificial sequence
<220>
   <223> Synthetic peptide
<220>
   <221> MISC_FEATURE
   <222> (11) .. (11)
   <223> Azetidine-2-carboxylic acid
<400> 74
   Asp Thr Asp Leu Asp Ala Glu Ala Thr Ala Xaa Tyr Ile Pro Ala Asp
   1               5                   10                  15
   Asp Asp Phe Gln
               20
<210> 75
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 75
   Asp Thr Asp Leu Asp Ala Glu Ala Ile Ala Xaa Tyr Ile Pro Ala Asp
   1               5                   10                  15
   Asp Asp Phe Gln
               20
<210> 76
   <211> 20
   <212> PRT
   <213> Artificial sequence
<220>
   <223> Synthetic peptide
<220>
   <221> MISC_FEATURE
   <222> (11) .. (11)
   <223> Azetidine-2-carboxylic acid
<400> 76
   Asp Thr Asp Leu Asp Ile Glu Ala Val Ala Xaa Tyr Ile Pro Ala Asp
   1               5                   10                  15
   Asp Asp Phe Gln
               20
<210> 77
   <211> 20
   <212> PRT
   <213> Artificial sequence
<220>
   <223> Synthetic peptide
<220>
   <221> MISC_FEATURE
   <222> (11).. (11)
   <223> Azetidine-2-carboxylic acid
<400> 77
   Asp Thr Asp Leu Asp Val Glu Ala Val Ala Xaa Tyr Ile Pro Ala Asp
   1               5                   10                  15
   Asp Asp Phe Gln
               20
<210> 78
   <211> 20
   <212> PRT
   <213> Artificial sequence
<220>
   <223> Synthetic peptide
<220>
   <221> MISC_FEATURE
   <222> (11)..(11)
   <223> Azetidine-2-carboxylic acid
<400> 78
   Asp Thr Asp Leu Asp Thr Glu Ala Val Ala Xaa Tyr Ile Pro Ala Asp
   1               5                   10                  15
   Asp Asp Phe Gln
               20
<210> 79
   <211> 20
   <212> PRT
   <213> Artificial sequence
<220>
   <223> Synthetic peptide
<400> 79
   Asp Thr Asp Leu Asp Leu Glu Met Leu Lys Pro Tyr Ile Pro Met Asp
   1               5                   10                  15
   Asp Asp Phe Gln
               20
<210> 80
   <211> 20
   <212> PRT
   <213> Artificial sequence
<220>
   <223> Synthetic peptide
<400> 80
<210> 81
   <211> 20
   <212> PRT
   <213> Artificial sequence
<220>
   <223> Synthetic peptide
<400> 81
<210> 82
   <211> 20
   <212> PRT
   <213> Artificial sequence
<220>
   <223> Synthetic peptide
<400> 82
<210> 83
   <211> 20
   <212> PRT
   <213> Artificial sequence
<220>
   <223> Synthetic peptide
<400> 83
<210> 84
   <211> 20
   <212> PRT
   <213> Artificial sequence
<220>
   <223> Synthetic peptide
<400> 84
<210> 85
   <211> 20
   <212> PRT
   <213> Artificial sequence
<220>
   <223> Synthetic peptide
<400> 85
   Asp Thr Asp Leu Asp Leu Glu Met Leu Glu Pro Tyr Ile Pro Met Asp
   1 5 10 15
   Asp Asp Phe Gln
   20
<210> 86
   <211> 20
   <212> PRT
   <213> Artificial sequence
<220>
   <223> Synthetic peptide
<400> 86
<210> 87
   <211> 20
   <212> PRT
   <213> Artificial sequence
<220>
   <223> Synthetic peptide
<400> 87
<210> 88
   <211> 20
   <212> PRT
   <213> Artificial sequence
<220>
   <223> Synthetic peptide
<400> 88
   Asp Thr Asp Leu Asp Leu Glu Met Leu Leu Pro Tyr Ile Pro Met Asp
   1 5 10 15
   Asp Asp Phe Gln
   20
<210> 89
   <211> 20
   <212> PRT
   <213> Artificial sequence
<220>
   <223> Synthetic peptide
<400> 89
   Asp Thr Asp Leu Asp Tyr Glu Met Ile Ala Pro Tyr Ile Pro Ala Asp
   1 5 10 15
   Asp Asp Phe Gln
   20
<210> 90
   <211> 20
   <212> PRT
   <213> Artificial sequence
<220>
   <223> Synthetic peptide
<400> 90
   Asp Thr Asp Leu Asp Trp Glu Met Val Ser Pro Tyr Ile Pro Ala Asp
   1 5 10 15
   Asp Asp Phe Gln
   20
<210> 91
   <211> 20
   <212> PRT
   <213> Artificial sequence
<220>
   <223> Synthetic peptide
<400> 91
   Asp Thr Asp Leu Asp Trp Glu Ala Val Ser Pro Tyr Ile Pro Ala Asp
   1 5 10 15
   Asp Asp Phe Gln
   20
<210> 92
   <211> 20
   <212> PRT
   <213> Artificial sequence
<220>
   <223> Synthetic peptide
<400> 92
   Asp Thr Asp Leu Asp Trp Glu Met Val Ala Pro Tyr Ile Pro Ala Asp
   1 5 10 15
   Asp Asp Phe Gln
   20

## Description

### FIELD OF THE INVENTION

The present invention provides methods for measuring EGLN activity, and use of the methods to identify agents which modulate EGLN activity.

### BACKGROUND

The 2-oxoglutarate dioxygenase enzymes are responsible for various physiological processes associated with normal cellular maintenance and cellular response to a changing environment and stress. The 2-oxoglutarate dioxygenases are non-heme-Fe(U)-dependent oxygenases that modify, e.g., by hydroxylation, various substrates. In addition to iron, the 2-oxoglutarate dioxygenases require oxygen, 2-oxoglutarate, and ascorbic acid for their activity. Some of the best-studied family members include the collagen modifying enzymes lysine hydroxylase (EC 1.14.11.4), prolyl 3-hydroxylase (EC 1.14.11.7), and the α-subunit of prolyl 4-hydroxylase (P4H; EC 1.14.11.2). (See, e.g., Majamaa et al. (1985) Biochem J 229:127-133; Myllyharju and Kivirikko (1997) EMBO J 16:1173-1180.)

All of the 2-oxoglutarate dioxygenase enzymes utilize a common catalytic mechanism that involves coordination of 2-oxoglutarate and dioxygen to enzyme-bound iron. The oxygen is subsequently cleaved, and one atom of oxygen is transferred to 2-oxoglutarate to produce carbon dioxide and succinate. The remaining Fe-bound atom of oxygen then modifies a second substrate; in the case of P4H, a proline residue residing within a particular amino acid sequence framework of collagen is oxidized to hydroxyproline. Thus, the enzymes of this family require iron, use 2-oxoglutarate and dioxygen as substrates, and produce succinate and carbon dioxide as products. The additional substrate utilized by each enzyme differs between family members, and thereby distinguishes the various members of the family and provides a unique context for each reaction catalyzed. The enzymes also require ascorbic acid as a cofactor to prevent enzyme inactivation.

Egl-9 is the founding member of a newly described group of 2-oxoglutarate dioxygenases. (Aravind and Koonin (2001) Genome Biol 2:RESEARCH0007; Taylor (Taylor (2001) Gene 275:125-132.) Egl-9, originally identified as a gene product required for egg laying in *Caenorhabditis elegans,* was also found to be required for *Pseudomonas aeruginosa*-induced muscle paralysis in the nematode. Additionally, a rat homolog, SM-20, expressed in tissues and cells derived from muscle (smooth, skeletal, and cardiac) and nerve, was identified. (Darby et al. (1999) Proc Natl Acad Sci USA 96:15202-15207; also, see Wax et al. (1994) J Biol Chem 269(17):13041-13047; Wax et al. (1996) Lab Invest 74(4):797-808.) Interestingly, Egl-9 homologs have also been identified in bacteria, e.g., *Pseudomonas aeruginosa* and *Vibrio cholera,* as well as in Drosophila, suggesting evolutionary conservation of the Egl-9 enzyme group. (See, e.g., Aravind and Koonin, *supra.*) The Egl-9 family has been further expanded to include human and mouse homologs, identified as EGLN1, EGLN2, and EGLN3. (Taylor, *supra.*)

Although the substrate of the EGLN family was not known, these enzymes were associated with regulation of cell survival and growth in response to various factors. (See, e.g., Lipscomb et al. (1999) J Neurochem 73:429-432; Moschella et al. (1999) Gene Expr 8:59-66; and Lipscomb et al. (2001) J Biol Chem 276:5085-5092.) Recognition of the EGLN substrate as the alpha subunit of hypoxia inducible factor (HIFα) has implicated this enzyme family in cellular response to oxygen level. (Epstein et al. (2001) Cell 107:43-54; Bruick and McKnight (2001) Science 294:1337-1340.) HIF, a transcription factor that activates gene transcription under conditions of low oxygen, is a heterodimer composed of a single beta (HIFβ/ARNT) subunit and a family of HIFα subunits. All cells appear to express HIF-1α and HIF-1β constitutively; however, under normoxic conditions the HIFα subunit is hydroxylated by EGLN and is subsequently degraded by the ubiquitin ligase system. Under low oxygen conditions, the HIFα subunit is stable and able to accumulate within the cell, where it dimerizes with HIFβ, translocates to the nucleus, and initiates gene transcription. The specific genes transcribed by the HIF transcription factor provide compensatory local and systemic responses that facilitate both cell survival and metabolic recovery during hypoxic events.

Several publications report investigation of the peptide framework within the HIFα substrate that is essential for oxygen sensitivity. Investigations originally focused on the domain within HIFα necessary for interaction with pVHL. (See, e.g., Huang et al. (1998) Proc Natl Acad Sci USA 95:7987-7992; Tanimoto et al. (2000) EMBO J 19:4298-4309; and Poellinger et al., International Publication No. WO 02/12326.) Although these studies identified a basic motif necessary for recognition of HIFα by pVHL, they did not determine the amino acid sequence requirements for interaction of HIFα with EGLN. More recently, investigations have focused on the domain within HIFα necessary for hydroxylation by EGLN. (See, e.g., Hirsila et al. (2003) J Biol Chem 278:30772-30780; and Huang et al. (2002) J Biol Chem 277:39792-39800.) Although this domain overlaps with, the pVHL interacting domain, the specific amino acid framework appears to show different structural requirements.

Huang et al (2002) J Biol Chem 277(42): 39792-800 discloses sequence determinants in HIF-1α for hydroxylation by the prolyl hydroxylases PHD1, PHD2, and PHD3.

WO 03/049686 discloses a screening assay for compounds that inhibit HIF-specific prolyl hydroxylase activity and thereby stabilize HIFα. The assay involves the measurement of hydroxylation of a HIF peptide, such as [methoxycoumarin]-DLDLEALAPYIPADDDFQL-amide.

Oehme et al (2004) Anal Biochem 330(1): 74-80 discloses a nonradioactive 96-well plate assay for the detection of hypoxia-inducible factor prolyl hydroxylase activity.

WO 02/074981 relates to a class of hydroxylases and variants and fragments thereof having HIF hydroxylation activity. The polypeptides are said to have in particular prolyl hydroxylase activity. An assay method monitors the interaction of the HIF hydroxylase with a substrate.

A method for measuring EGLN activity, especially in a high-throughput formal, would be advantageous for the study of enzyme kinetics and screening and design of enzyme inhibitors. Additionally, identification of substrate recognition motifs that showed preference for one EGLN family member relative to other family members would provide insight into inhibitor design for targeting individual family members. The present invention provides such a method, and defines substrates showing selectivity in interaction with various EGLN family members. The present invention also provides use of the methods for identifying agents that modulate activity of an EGLN enzyme.

### SUMMARY OF THE INVENTION

The present invention provides methods of measuring the activity of an EGLN enzyme. In one aspect, the invention provides the method according to claim 1. In various embodiments, the 2-oxoglutarate is provided in a range of from about 0.1 to 100 µM, e.g., from 10 to 100 µM; and may be supplied in any suitable form including, e.g., a salt.

In another aspect, the invention provides the method according to claim 2.

In various embodiments, the 2-oxoglutarate is provided in a range of from about 0.1 to 100 µM, e.g., from 10 to 100 µM; and may be supplied in any suitable form including, e.g., a salt.

In various embodiments, the EGLN enzyme used in the methods of the invention comprises the sequence of SEQ ID NO:1. In other embodiments, the EGLN enzyme comprises the sequence of SEQ ID NO:3. In particular embodiments, the EGLN enzyme further comprises the sequence of SEQ ID NO:2. In various aspects, the EGLN enzyme is selected from the group consisting of EGLN1, EGLN2, EGLN3, and active fragments of EGLN1, EGLN2, and EGLN3. The enzymes of these embodiments and aspects may be obtained from any species, or may be produced using an EGLN enzyme expressed from a recombinant polynucleotide encoding the EGLN enzyme or a functional fragment thereof.

In various aspects, the peptide used in the methods of the invention is at least about 10 amino acids in length, more particularly at least about 15 amino acids in length, and even more particularly at least about 20 amino acids in length. In various embodiments, the peptide comprises a sequence selected from the group consisting of SEQ ID NOs:62-78. In particular embodiments, the peptide is 20 amino acids in length and is selected from the group consisting of SEQ ID NOs:62-78. In various aspects, the peptides are composed of amino acids that include both L- and D-isomers. In other aspects, the peptides are composed of L-amino acid isomers. In some embodiments, Z is proline and is a D-isomer. In other embodiments, Z is selected from the group consisting of L- and D-isomers of azedidine-2-carboxylate, 3,4-dehydroproline, and b-thioproline. In particular embodiments, Z is selected from L-isomers azedidine-2-carboxylate, 3,4-dehydroproline, and b-thioproline.

In one embodiment, the methods of the invention comprise combining an enzyme selected from the group consisting of EGLN1, an active fragment of EGLN1, EGLN2, and an active fragment of EGLN2. In various embodiments therein, the peptide for use in the method comprises the sequence X₁-X₂-X₃-X₄-X₅-Z-X₆, wherein X₁ is selected from tyrosine, tryptophan, methionine, isoleucine, phenylalanine, aspartate, alanine, glutamate, cysteine, proline, glycine, and leucine; X₂, and X₃ are independently selected from any amino acid; X₄ is selected from isoleucine, valine, arginine, phenylalanine, tyrosine, methionine, threonine, lysine, tryptophan, cysteine, asparagine, histidine, serine, alanine, glycine, glutamate, glutamine, or leucine; X₅ is selected from threonine, serine, lysine, glutamine, methionine, or alanine; X₆ is selected from phenylalanine or tyrosine; and Z is a proline analog that is not susceptible to hydroxylation by HIF prolyl hydroxylase, e.g. azetidine-2-carboxylate, 3,4-dehydroproline, or b-thioproline. In various embodiments, the 2-oxoglutarate is provided in a range of from about 0.1 to 100 µM, e.g., from 10 to 100 µM; and may be supplied in any suitable form including, e.g., a salt. In particular embodiments, the peptide comprises a sequence selected from the group consisting of SEQ ID NOs: 62 and 63. In specific embodiments, the peptide is selected from the group consisting of SEQ ID NOs: 62 and 63. In some embodiments, a test agent is included in the assay, and the ability of the test agent to modulate activity of the enzyme is determined.

In another embodiment, the methods of the invention comprise combining an enzyme selected from EGLN3 or an active fragment of EGLN3. In various embodiments, the 2-oxoglutarate is provided in a range of from about 0.1 to 100 µM, e.g., from 10 to 100 µM; and may be supplied in any suitable form including, e.g., a salt. In particular embodiments, the peptide comprises a sequence selected from the group consisting of SEQ ID NOs: 62, 63, 69 and 72. In specific embodiments, the peptide is selected from the group consisting of SEQ ID NOs: 62, 63, 69 and 72. In some embodiments, a test agent is included in the assay, and the ability of the test agent to modulate activity of the enzyme is determined.

The methods of the invention comprise a reducing agent. The reducing agent may be selected from the group consisting of, but not limited to, ascorbate and potassium ferrocyanide. The reducing agent may be provided in a range of from about 0.1 to 10 mM, e.g., from 0.5 to 5 mM; and may be supplied in any suitable form including, e.g., a salt, e.g., sodium ascorbate, potassium ferrocyanide, etc. The methods may also comprise iron, e.g., ferrous ions provides as ferrous chloride, ferrous sulfate, etc. In various embodiments, ferrous ions are provided in a range of from about 0 to 500 µM, e.g., from 25 to 250 µM, and in particular from 50 to 200 µM.

In some embodiments, Z is azetidine-2-carboxylate. In particular embodiments therein, the peptide comprises a sequence selected from the group consisting of SEQ ID NOs:63, 64, 65, 66, 67, 68, 69, 70, 71, 73, 74, 75, 76, 77, and 78. In specific embodiments, the peptide is selected from the group consisting of SEQ ID NOs:63, 64, 65, 66, 67, 68, 69, 70, 71, 73, 74, 75, 76, 77, and 78. In other embodiments, Z is 3,4-dehydroproline. In particular embodiments therein, the peptide comprises the sequence of SEQ ID NO:62. In a specific embodiment, the peptide is SEQ ID NO:62. In still other embodiments, Z is b-thioproline. In particular embodiments therein, the peptide comprises the sequence of SEQ ID NO:72. In a specific embodiment, the peptide is SEQ ID NO:72.

In various embodiments of the present invention, measuring EGLN activity comprises measuring carbon dioxide produced by the reaction of the EGLN enzyme on the 2-oxoglutarate substrate, wherein the amount of carbon dioxide produced is directly proportional to the activity of the EGLN enzyme. In other embodiments, measuring EGLN activity comprises measuring conversion of the reducing agent to its oxidized form during the reaction, wherein the amount of reducing agent that is oxidized is directly proportional to the activity of the EGLN enzyme.

These and other embodiments of the subject invention will readily occur to those of skill in the art in light of the disclosure herein, and all such embodiments are specifically contemplated.

### BRIEF DESCRIPTION OF THE FIGURES

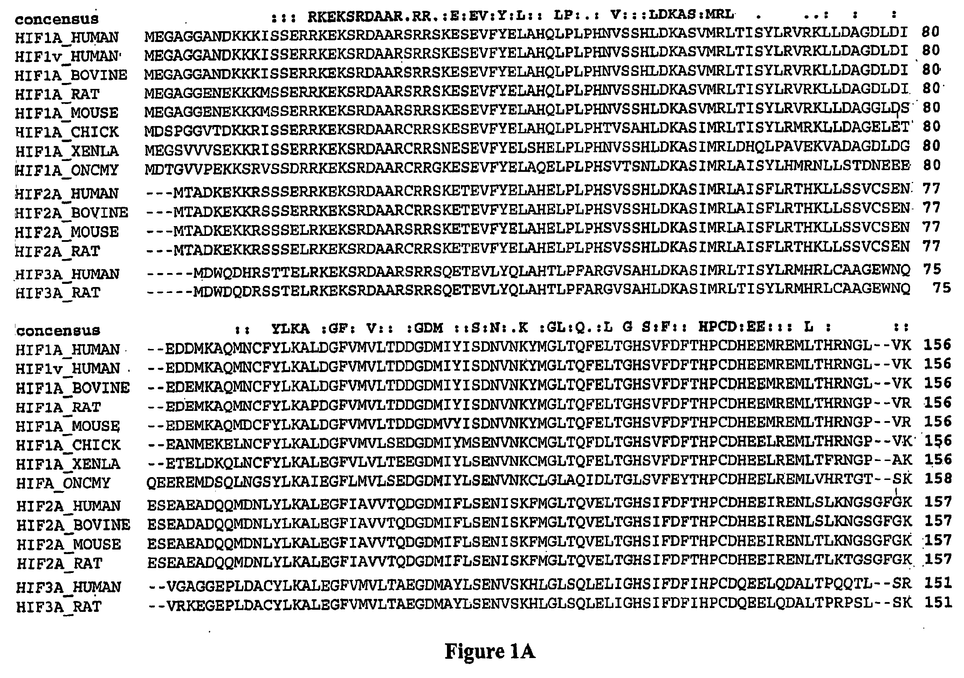

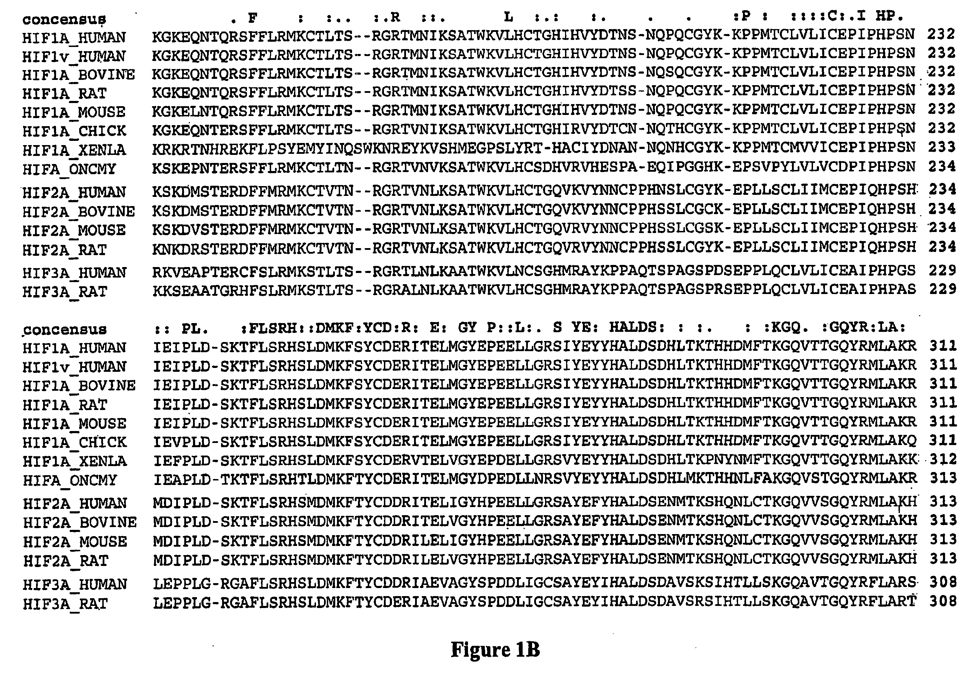

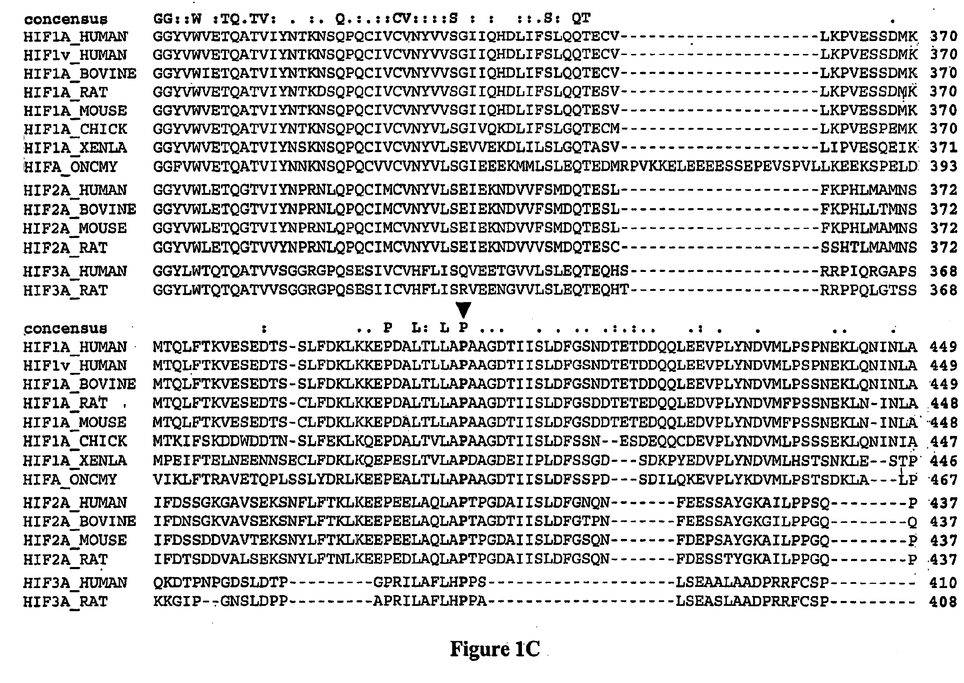

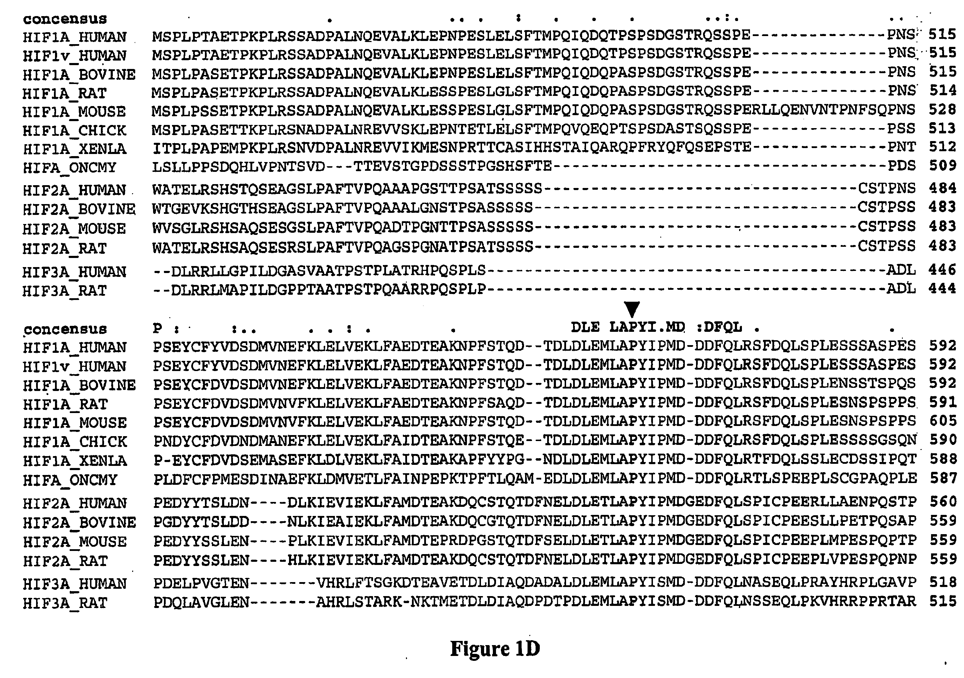

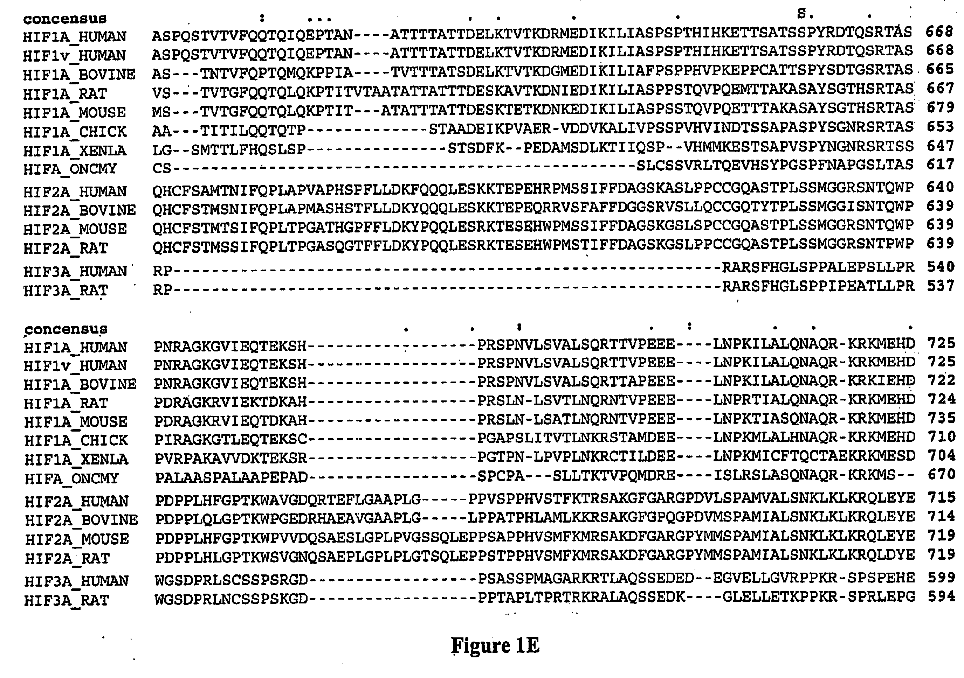

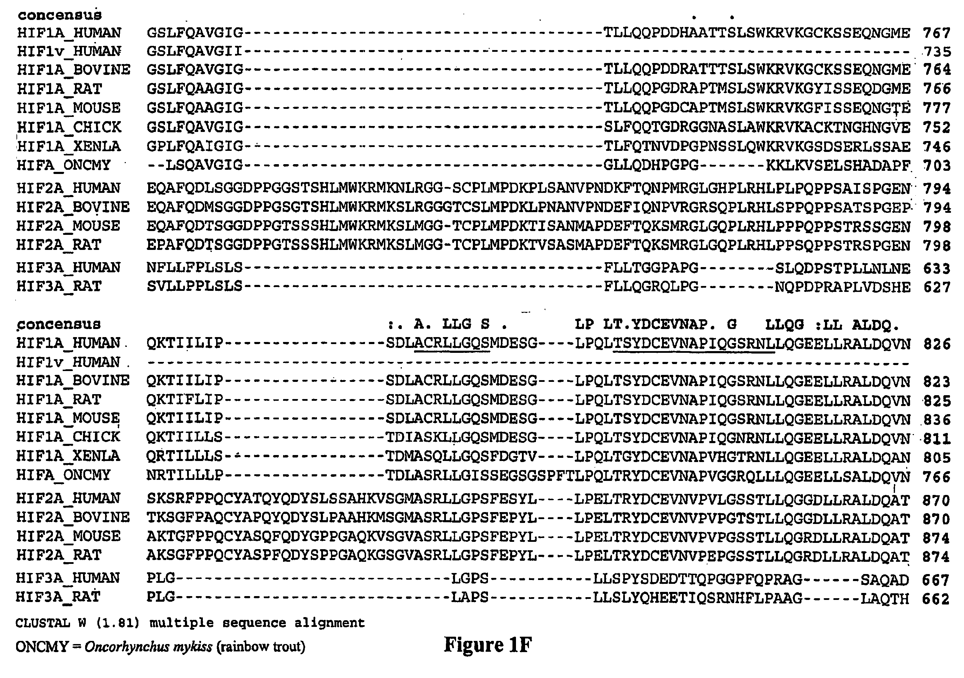

Figure 1 (1A, 1B, 1C, 1D, 1E, and IF) shows an alignment of HIFα subunits from various species. The alignment was generated using the CLUSTALW program (version 1.81; Thompson et al. (1994) Nucleic Acids Res 22:4673-4680).

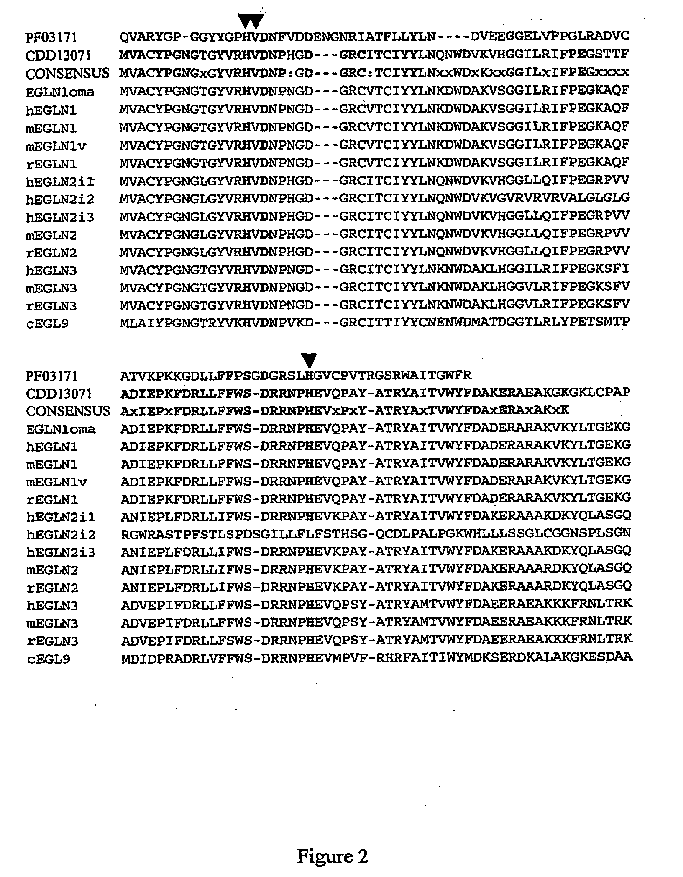

Figure 2 shows an alignment of the active domain of various EGLN family members. The alignment was generated using the CLUSTALW program (v.1.81; Thompson et al., *supra*)*.* In the figure, PF03171 denotes a 2-oxoglutarate Fe(II) oxygenase superfamily motif identified by multiple sequence alignment and Hidden Markov Model (HMM) analysis as generated by the PFAM database (version 13.0; Bateman et al. (2002) Nucleic Acids Research 30(1):276-280); and CDD13071 denotes an Egl-9 proline hydroxylase domain identified by multiple sequence alignment as generated by the Conserved Domain Database (CDD, version 2.00; Marchler-Bauer et al. (2003) Nucleic Acids Res 31:383-387).

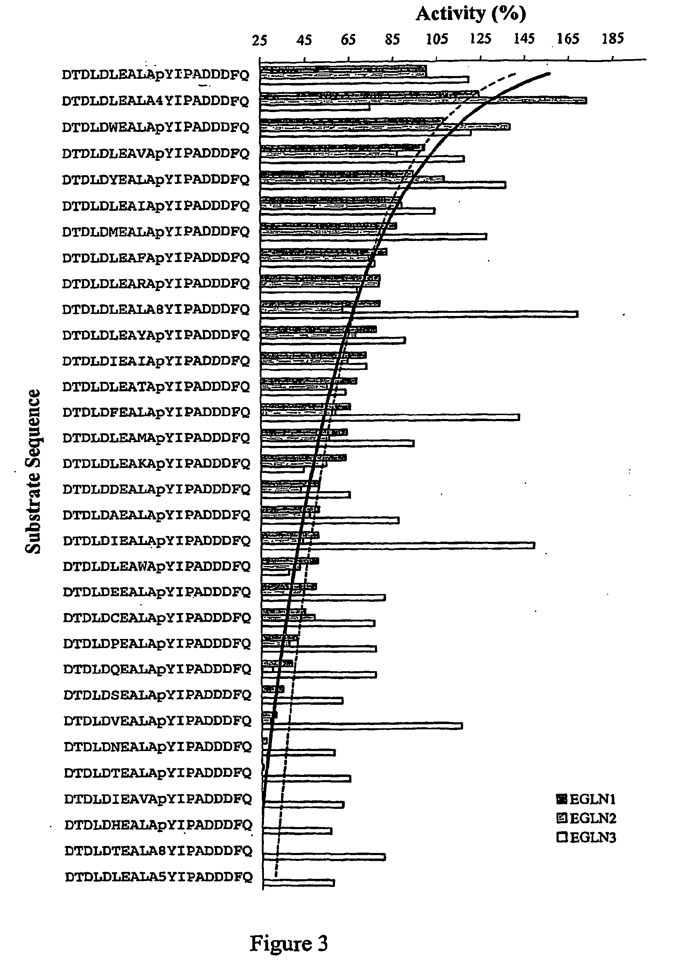

Figure 3 shows a plot of substrate peptide activity relative to a control peptide ordered with respect to EGLN1. Trend lines in the figure show activity relative to EGLN1 (solid line) and EGLN2 (dashed line).

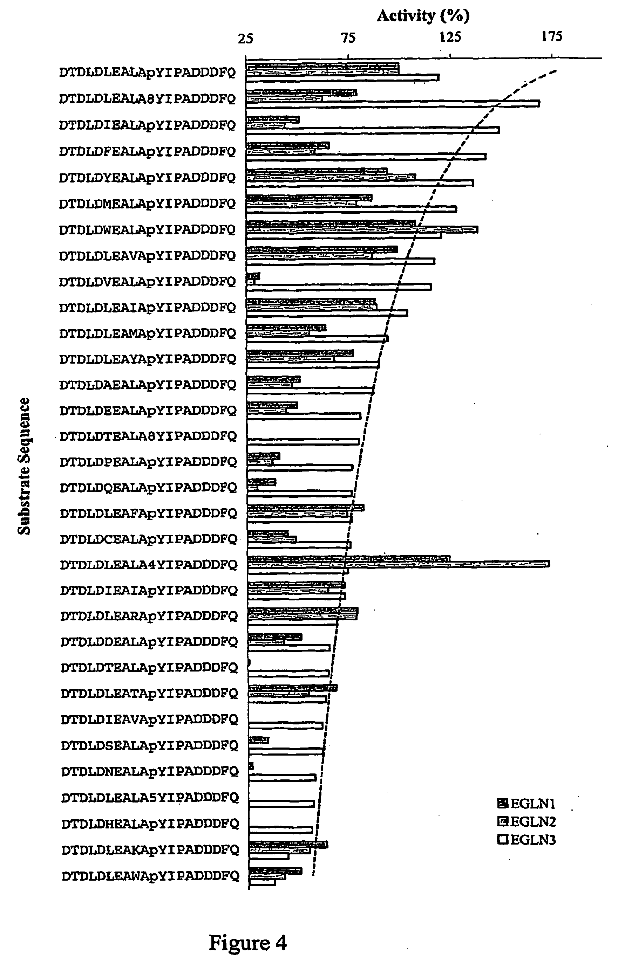

Figure 4 shows a plot of substrate peptide activity relative to a control peptide ordered with respect to EGLN3. The trend line in the figure shows activity relative to EGLN3.

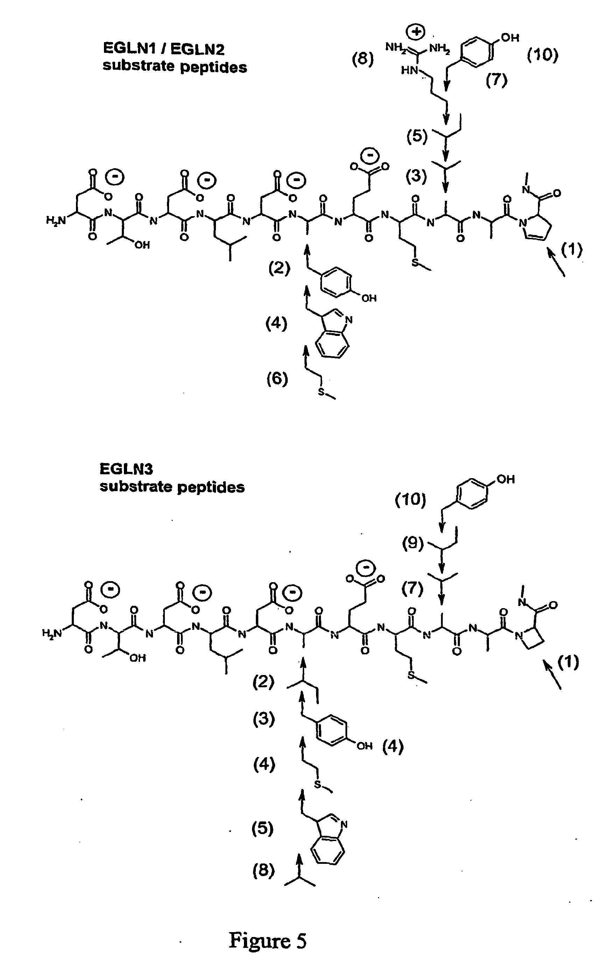

Figure 5 shows various modifications to the basic substrate structure that can be made while preserving enzyme activity. The panel at the top illustrates modifications to the proline residue (1) and additional amino acids (2 to 10) that preserve suitable activity of EGLN1 and EGLN2. The panel at the bottom illustrates modifications to the proline residue (1) and additional amino acids (2 to 10) that preserve suitable activity of EGLN3. Only a portion of each peptide sequence is illustrated in the figure.

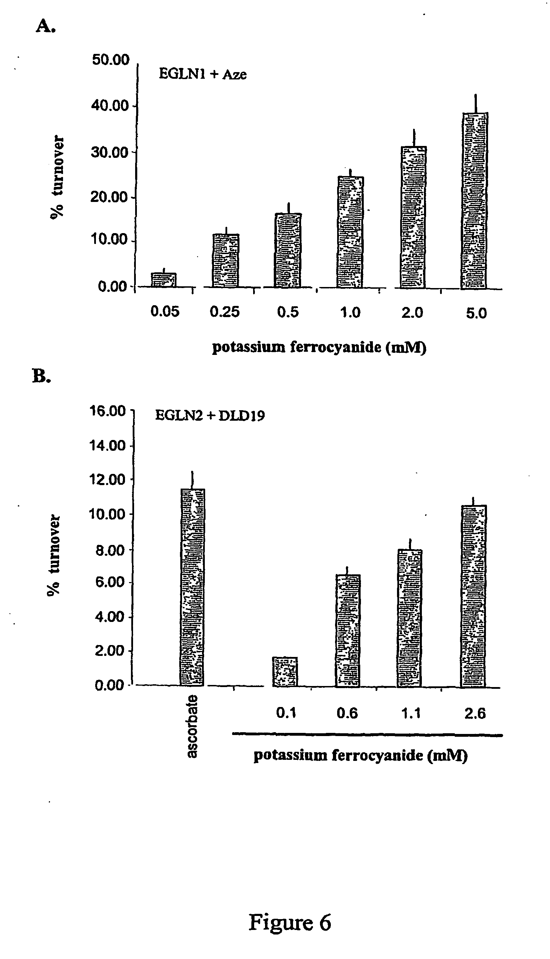

Figure 6 (6A and 6B) shows percent turnover of 2-oxoglutarate relative to various concentrations of potassium ferrocyanide. Figure 6A shows activity of EGLN1 activity in the presence of an azetidine-containing peptide according to the modified reaction (II). Each data point represents the average of three experiments. Figure 6B shows activity of EGLN2 in the presence of DLD19 peptide. Reaction in the presence of 1mM ascorbate was carried out as a control. Each data point represents the average of three experiments.

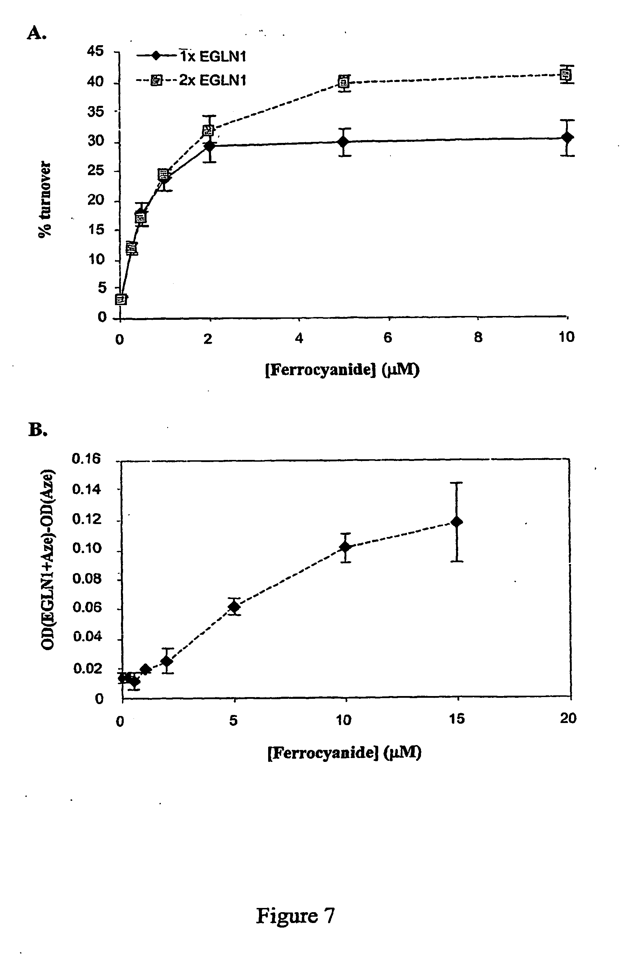

Figure 7A shows the percent turnover of 2-oxoglutarate relative to potassium ferrocyanide concentration using two different concentrations of EGLN1. Figure 7B shows rate of formation of ferricyanide product relative to initial ferrocyanide concentration in modified reaction (II). The formation of ferricyanide was monitored by following optical density of Fe³⁺ at a wavelength of 405 nm. The optical density of reactions without enzyme was subtracted from OD of reactions with enzyme to correct for non-enzymatic formation of product.

### DETAILED DESCRIPTION OF THE INVENTION

Before the present methods are described, it is to be understood that the invention is not limited to the particular reagents described, as these may vary. It is also to be understood that the terminology used herein is intended to describe particular embodiments of the present invention, and is in no way intended to limit the scope of the present invention as set forth in the appended claims.

It must be noted that as used herein and in the appended claims, the singular forms "a," "an," and "the" include plural references unless context clearly dictates otherwise. Thus, for example, a reference to "a peptide" may include a plurality of such peptides and to equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described.

The practice of the present invention will employ, unless otherwise indicated, conventional methods of chemistry, biochemistry, molecular biology, cell biology, genetics, etc., within the skill of the art. Such techniques are explained fully in the literature.

### INVENTION

The present invention provides a method for measuring the activity of various EGLN enzymes utilizing heretofore-unknown substrates, which show a range of specificity and variability for EGLN isoforms, and novel reaction parameters. Although the substrates disclosed herein share some similarities with the HIFα polypeptide, they contain substantial and non-obvious changes in the substrate structure and provide unique assay conditions, enzyme kinetics, and assay readouts. The assays are useful to more fully characterize substrate-enzyme specificity, and to screen and identity agents that modify the ability of specific EGLN enzymes to interact with their respective substrates.

In one aspect, the invention provides the method of claim 1. Conditions suitable for EGLN enzyme activity generally include the presence of oxygen, and may optionally include added iron. In some embodiments, the reducing agent is ascorbic acid, whereas in other embodiments the reducing agent is potassium ferrocyanide. In certain aspects, the peptide substrate shows similar specificity for the various EGLN enzymes. In other aspects, the peptide substrate shows higher specificity for one EGLN enzyme relative to other EGLN family members. In specific embodiments, the peptide substrate shows higher specificity for EGLN1 and EGLN2 than for EGLN3. Alternatively, in other specific embodiments, the peptide substrate shows higher specificity for EGLN3 than for EGLN1 and EGLN2. In still other embodiments, the invention contemplates peptide substrates that show higher affinity for, e.g., EGLN1 than for EGLN2, or for EGLN2 than for EGLN1.

In another aspect, the invention provides the method of claim 2.

### EGLN

EGLN are 2-oxoglutarate dioxygenase enzymes that, among other potential substrates, are known to hydroxylate specific proline residues in HIFα proteins. The proline residues hydroxylated by EGLN include the proline residues that occur in the human HIF-1α native sequence at residues 402 (P₄₀₂) and 564 (P₅₆₄), and corresponding proline residues in HIFα subunits obtained from another species as shown, e.g., in Figure 1. In preferred embodiments, the EGLN utilized in the assay is selected from members of the Egl-9 enzyme family described by Taylor (*supra*)*,* and characterized by Aravind and Koonin (*supra*), Epstein et al. (*supra*), and Bruick and McKnight (*supra*). In some embodiments of the present invention, the EGLN enzyme may be selected from an isoform of EGLN1, EGLN2, and/or EGLN3. The EGLN1 isoform may be selected from the group that includes, but is not limited to, human EGLN1 (hEGLN1, GenBank Accession No. AAG33965; Dupuy et al. (2000) Genomics 69:348-54), mouse EGLN1 (GenBank Accession No. CAC42515), and rat EGLN1 (GenBank Accession No. P59722); the EGLN2 isoform may be selected from the group that includes, but is not limited to, human EGLN2 isoform 1 (GenBank Accession No. CAC42510; Taylor, *supra*), human EGLN2 isoform 3 (GenBank Accession No. NP_542770), mouse EGLN2 (GenBank Accession No. CAC42516), and rat EGLN2 (GenBank Accession No. AA046039); and the EGLN3 isoform may be selected from the group that includes, but is not limited to, human EGLN3 (GenBank Accession No. CAC42511; Taylor, *supra*)*,* mouse EGLN3 (GenBank Accession No. CAC42517); and rat EGLN3 (SM-20) (GenBank Accession No. AAA19321). In other embodiments of the present invention, EGLN may include *Caenorhabditis elegans* EGL-9 (GenBank Accession No. AAD56365) and *Drosophila melanogaster* CG1114 gene product (GenBank Accession No. AAF52050).

In other embodiments, the EGLN utilized in the assays of the present invention may comprise an active fragment of any of the EGLN enzyme family members described above or known to those skilled in the art. An active fragment would preferably comprise a domain containing the sequence

H-X-D-[X]ₙ-H

where X is any amino acid and n is from about 40 to 80, preferably from about 50 to 65, and most preferably about 58. (See, e.g., Figure 2.) For example, an active fragment of a HIF PH utilized in the assay may comprise the consensus sequence defined by the Protein Family (PFAM) database accession number 3171 (SEQ ID NO: 1), the consensus sequence defined by the Entrez Conserved Domain Database (CDD) accession number 21489 (SEQ ID NO:2), or the consensus sequence shown in Figure 2 (SEQ ID NO:3). (Bateman et al. (2004) Nucleic Acids Res 32(1):D138-141; Marchler-Bauer et al. (2003) Nucleic Acids Res 31:383-387.)

The enzyme used in the present invention may be endogenously produced by a cell, wherein the cell is originally obtained from a natural source such as a single celled or multicellular eukaryotic organism. For example, the enzyme may be obtained by digestion of whole organism, such as a nematode, e.g., *Caenorhabditis elegans,* or insect, e.g., *Drosophila melanogaster,* digestion of isolated organ or tissue, e.g., obtained from mouse or rat kidney, lung, liver, brain, etc; or lysation of a clonal cell population, e.g., an established cell line. Alternatively, the enzyme may be produced using recombinant DNA technology; e.g., the gene or transcript polynucleotide encoding the enzyme may be inserted into a host cell or organism, and the host may, constitutively or under specific conditions, express the protein encoded by the exogenous polynucleotide sequence.

Regardless of whether the enzyme is produced from endogenous or exogenous polynucleotides, the enzyme may be used in the present assay while contained within the intact host cells or within a cellular lysate produced from the cells. For example, a cell that endogenously produces the enzyme, such as hepatocytes, e.g., Hep3B cells; endothelial cells, e.g. human microvascular endothelial cells (HMVEC); fibroblasts, e.g., human foreskin fibroblasts; etc. can be grown in culture to a suitable density, and cell lysates can then be prepared using standard techniques known by those of skill in the art. Cells may be chosen by their endogenous expression of specific EGLNs; e.g., specific expression of EGLN-1 was recently reported under normoxic conditions in arterial endothelial cells. (See, e.g., Takahashi et al. (2004) Biochem Biophys Res Comm 317:84-91.) Alternatively, expression of EGLNs may be induced, e.g., by growing cells for a suitable period of time under low oxygen, i.e., hypoxic, conditions. Such conditions are generally known to those of skill in the art.

Optionally, the enzyme may be partially purified or concentrated by, e.g., fractionation of the lysate containing the enzyme. Alternatively, the enzyme may be purified from other components in the lysate using any method known by those of skill in the art, e.g., by polyacrylamide gel electrophoresis (PAGE) or affinity chromatography. Enzyme, and particularly active fragments of the enzyme, may also be synthesized chemically utilizing, e.g., FMOC chemistry performed using, e.g., an automated 432A peptide synthesizer (Applied Biosystems, Inc., Foster City CA), or other technologies known to those skilled in the art.

### PEPTIDES

The present application discloses peptides that show a range of specificity and variability for EGLN isoforms. Although these peptides share some similarities with the HIFα polypeptide, they contain substantial and non-obvious changes to substrate structure and provide unique assay conditions and enzyme kinetics.

As used herein, the term "HIFα" refers to the alpha subunit of hypoxia inducible factor protein. HIFα occurs in three general forms, HIF-1α, HIF-2a, and HIF-3α. Further, each general form may exist within an organism in different isoforms; e.g., HIF-1α includes, but is not limited to, human HIF-1α isoform 1 (Genbank Accession No. Q16665; HIF1A_HUMAN) and isoform 2 (Genbank Accession No. NP_851397; HIF1v_HUMAN), murine H1F-1α (Genbank Accession No. Q61221; HIF1A_MOUSE), rat HIF-1α (Genbank Accession No. CAA70701; HIF1A_RAT), and cow HIF-1α (Genbank Accession No. BAA78675; HIF1A_BOVINE). Similarly, HIF-2α includes, but is not limited to, human HIF-2α (Genbank Accession No. AAB41495; HIF2A_HUMAN), mouse HIF-2α (Genbank Accession No. BAA20130 and AAB41496; HIF2A_MOUSE), rat HIF-2α (Genbank Accession No. CAB96612; HIF2A_RAT), and bovine HIF-2α (Genbank Accession No. BAA78676; HIF2A_BOVINE); and HIF-3α includes, but is not limited to, human HIF-3α (Genbank Accession No. AAD22668; HIF3A_HUMAN), mouse HIF-3α (Genbank Accession No. AAC72734), and rat HIF-3α (Genbank Accession No. CAB96611; HIF3A_RAT). Various non-mammalian HIFα proteins have also been described including, but not limited to, *Xenopus laevis* HIF-1α (Genbank Accession No. CAB96628; HIF1A_XENLA), *Drosophila melanogaster* HIF-1α (Genbank Accession No. JC4851), *Oncorhynchus mykiss* HIF-1α (Genbank Accession No. Q98SW2; HIF1A_ONCMY), and chicken HIF-1α (Genbank Accession No. BAA34234; HIF1A_CHICK).

Alignment of HIFα subunits, as shown in Figure 1, demonstrates conserved regions among the various family members. Conserved regions were originally analyzed for functional characteristics shared by HIFα subunits, such as their susceptibility to oxygen-regulated degradation. Deletion studies identified an oxygen degradation domain within the portion of HIFα defined by HIF-1α amino acid residues 401 to 603 (Huang et al., *supra).* An oxygen sensitive region was subsequently localized to amino acids 556 to 575 of HIF-1α, more specifically to a highly conserved colinear sequence corresponding to M₅₆₁LAPYIPM within HIF-1α(556-575). (Jiang et al. (1997) J Biol Chem 272:19253-19260; Tanimoto et al. (2000) EMBO J 19:4298-4309; Srinivas et al. (1999) Biochem Biophys Res Commun 260:557-561; and Ivan et al. (2001) Science 292:464-468.) Within this sequence, hydroxylation of the proline residue occurring in the context of the LXXLAP motif, e.g., as occurs in the HIF-1α native sequence at L₃₉₇TLLAP and L₅₅₉EMLAP and in corresponding portions of HIF-2α and HIF-3α, was found to be required for normoxic degradation of HIF-la. (See Ivan and Kaelin (2001) Science 292:464-468; Jaakkola et al. (2001) Science 292:468-472.) For example, regions corresponding to the C-terminal oxygen degradation domain (C-ODD) for HIF-1α, HIF-2α, and HIF-3α are shown below.
D₅₅₆LDLEMLAPYIPMD-DDFQLR (HIF-1α C-ODD; SEQ ID NO:4)
E₅₂₂LDLETLAPYIPMDGEDFQLS (HIF-2α C-ODD; SEQ ID NO:5)
A₄₈₁LDLEMLAPYISMD-DDFQLN (HIF-3α C-ODD; SEQ ID NO:6)

These sequences thus represent endogenous substrates for the HIF-PH enzymes. Further, residues shown in bold are conserved between the various HIFα isoforms and thereby may define a consensus sequence for EGLN hydroxylation.

The present application discloses further characterization of the substrate requirements of the EGLN enzyme family. In one aspect, the present application discloses a peptide comprising the sequence X₁-X₂-X₃-X₄-X₅-Z-X₆, wherein X₁, X₂, and X₃ are independently selected from any amino acid; X₄ is selected from isoleucine, valine, arginine, phenylalanine, tyrosine, methionine, threonine, lysine, tryptophan, cysteine, asparagine, histidine, serine, alanine, glycine, glutamate, glutamine, or leucine; X₅ is selected from threonine, serine, lysine, glutamine, methionine, isoleucine, arginine, histidine, glutamate, phenylalanine, cysteine, leucine, or alanine; X₆ is selected from phenylalanine or tyrosine; and Z is proline, with the proviso that X₁ and X₄ are not both leucine, or that X₄-X₅-Z-X₆ are not LAPY. Although peptides of any length may be used, the peptide is preferably at least about 10 nucleotides in length, particularly at least about 15, and more particularly at least about 20 nucleotides in length. Table 1 shows various examples of these peptides. The lowercase p in each peptide represents the proline that may be hydroxylated during the reaction.

**Table 1**

| **SEQ ID NO** | **SEQUENCE** | **Activity (%)*** | | |
|---|---|---|---|---|
| | | **EGLN1** | **EGLN2** | **EGLN3** |
| 7 | DTDLDLEMLApYIPMDDDFQ* | 100.00 | 100.00 | 119.43 |
| 8 | DTDLDWBALApYIPADDDFQ | 107.81 | 138.08 | 120.36 |
| 9 | DTDLDLEAVApYIPADDDFQ | 99.16 | 86.85 | 117.27 |
| 10 | DTDLDYEALApYIPADDDFQ | 94.17 | 108.03 | 136.12 |
| 11 | DTDLDLEALApYIPADDDFQ | 102.11 | 101.92 | 118.57 |
| 12 | DTDLDLEAIApYIPADDDFQ | 88.02 | 89.05 | 103.88 |
| 13 | DTDLDMEALApYIPADDDFQ | 86.40 | 79.06 | 127.54 |
| 14 | DTDLDLEAFApYIPADDDFQ | 82.17 | 73.87 | 76.42 |
| 15 | DTDLDLEARApYIPADDDFQ | 78.99 | 78.58 | 68.63 |
| 16 | DTDLDLEAYApYIPADDDFQ | 77.21 | 67.82 | 90.13 |
| 17 | DTDLDISAIApYIPADDDFQ | 72.54 | 64.29 | 72.83 |
| 18 | DTDLDLEATApYIPADDDFQ | 68.16 | 54.88 | 63.19 |
| 19 | DTDLDFEALApYIPADDDFQ | 65.22 | 58.57 | 142.09 |
| 20 | DTDLDLEAMApYIPADDDFQ | 63.67 | 55.82 | 94.29 |
| 21 | DTDLDLEAKApYIPADDDFQ | 63.19 | 54.56 | 44.13 |
| 22 | DTDLDDEALApYIPADDDFQ | 51.05 | 42.77 | 64.99 |
| 23 | DTDLDAEALApYIPADDDFQ | 51.03 | 46.86 | 87.15 |
| 24 | DTDLDIEALApYIPADDDFQ | 50.67 | 43.73 | 148.95 |
| 25 | DTDLDLEAWApYIPADDDFQ | 50.37 | 42.36 | 37.33 |
| 26 | DTDLDEEALApYIPADDDFQ | 49.63 | 44.26 | 80.78 |
| 27 | DTDLDLEANApYIPADDDFQ | 49.23 | 37.71 | 42.45 |
| 28 | DTDLDVEAIApYIPADDDFQ | 45-.33 | 36.60 | 43.26 |
| 29 | DTDLDCEALApYIPADDDFQ | 44.70 | 48.78 | 75.83 |
| 30 | DTDLDLEACApYIPADDDFQ | 41.31 | 40.40 | 49.41 |
| 31 | DTDLDPEALApYIPADDDFQ | 40.76 | 37.36 | 76.56 |
| 32 | DTDLDQEALApYIPADDDFQ | 38.74 | 30.09 | 76.44 |
| 33 | DTDLDGEALApYIPADDDFQ | 37.30 | 32.03 | 40.90 |
| 34 | DTDLDLEAAApYIPADDDFQ | 36.11 | 30.89 | 30.16 |
| 35 | DTDLDVEAYApYIPADDDFQ | 35.48 | 32.36 | 44.54 |
| 36 | DTDLDSEALApYIPADDDFQ | 34.66 | 23.84 | 61.30 |
| 37 | DTDLDVEAFApYIPADDDFQ | 34.10 | 37.45 | 44.20 |
| 38 | DTDLDAEAVApYIPADDDFQ | 34.01 | 26.29 | 35.57 |
| 39 | DTDLDLEAFiApYIPADDDFQ | 33.95 | 32.27 | 27.04 |
| 40 | DTDLDIEANApYIPADDDFQ | 33.21 | 34.41 | 30.45 |
| 41 | DTDLDVEALApYIPADDDFQ | 31.46 | 29.01 | 115.46 |
| 42 | DTDLDAEAIApYIPADDDFQ | 31.26 | 23.96 | 25.67 |
| 43 | DTDLDLEASApYIPADDDFQ | 29.57 | 31.60 | 25.14 |
| 44 | DTDLDNEALApYIPADDDFQ | 27.14 | 21.36 | 57.60 |
| 45 | DTDLDTEALApYIPADDDFQ | 25.67 | 20.49 | 64.55 |
| 45 | DTDLDTEALApYIPADDDFQ | 23.36 | 18.04 | 60.23 |
| 46 | DTDLDTEAYApYIPADDDFQ | 24.06 | 25.62 | 35.99 |
| 47 | DTDLDIEAVApYIPADDDFQ | 23.85 | 23.76 | 61.30 |
| 48 | DTDLDTEAIApYIPADDDFQ | 23.69 | 25.28 | 18.97 |
| 49 | DTDLDLEAQApYIPADDDFQ | 23.41 | 19.11 | 39.44 |
| 50 | DTDLDHSALApYIPADDDFQ | 23.35 | 21.47 | 55.84 |
| 51 | DTDLDVEAVApYIPADDDFQ | 22.81 | 16.64 | 48.95 |
| 52 | DTDLDTEAPApYIPADDDFQ | 22.18 | 28.49 | 24.15 |
| 53 | DTDLDTEATApYIPADDDFQ | 21.90 | 24.82 | 16.49 |
| 54 | DTDLDAEATApYIPADDDFQ | 20.84 | 19.40 | 18.52 |
| 55 | DTDLDVEATApYIPADDDFQ | 20.38 | 13.55 | 23.99 |
| 56 | DTDLDREALApYIPADDDFQ | 19.91 | 15.70 | 49.83 |
| 57 | DTDLDTEAVApYIPADDDFQ | 16.62 | 19.01 | 26.79 |
| 58 | DTDLDAEAAApYIPADDDFQ | 12.60 | 8.63 | 7.18 |
| 59 | DTDLDLEAGApYIPADDDFQ | 11.34 | 9.84 | 12.15 |
| 60 | DTDLDAEASApYIPADDDFQ | 10.49 | 8.82 | 4.11 |
| 61 | DTDLDLEAEApYIPADDDFQ | 9.97 | 13.24 | 16.93 |
| 79 | DTDLDLEMLKpYIPMDDDFQ | 68.69 | 57.72 | 74.49 |
| 80 | DTDLDLEMLQpYIPMDDFQ | 45.07 | 38.12 | 59.88 |
| 81 | DTDLDLEMLMpYIPIPMDDFQ | 26.43 | 27.81 | 58.93 |
| 82 | DTDLDLEMLIpYIPMDDDFQ | 15.56 | 17.82 | 100.70 |
| 83 | DTDLDLEMLRpYIPMDDDFQ | 19.78 | 14.34 | 77.56 |
| 84 | DTDLDLEMLHpYIPMDDDFQ | 2.41 | 3.47 | 68.22 |
| 85 | DTDLDLEMLEpYIPIMDDPQ | 2.27 | 3.35 | 52.77 |
| 86 | DTDLDLEMLFpYIPMDDDFQ | 4.16 | 0.63 | 59.34 |
| 87 | DTDLDLEMLCpYIPMDDDFQ | 1.94 | 0.40 | 63.11 |
| 88 | DTDLDLEMLLpYIPMDDDPQ | 2.36 | -0.42 | 46.19 |

| | | | | |
|---|---|---|---|---|
| *Control peptide DTD20; activity measures are relative to this peptide. | | | | |

Any polypeptide comprising one of these sequences, or any of the peptides as shown in Table 1, may be used in the methods disclosed herein. These peptides are used in a reaction additionally requiring co-substrate 2-oxoglutarate, an EGLN enzyme, and oxygen, as described below. The reaction may additionally include Fe²⁺. The reaction proceeds according to the standard reaction (I), in which 2-oxoglutarate substrate is converted to succinate and carbon dioxide and the unhydroxylated peptide substrate is hydroxylated.

The present application further demonstrates that the peptides show differential specificity for the various EGLN enzymes. Thus, the application discloses peptides optimized for EGLN1 and EGLN2, which as demonstrated herein, show similar specificity for the various peptides. For use in an assay wherein the enzyme is EGLN1 or EGLN2, or wherein it is desired that the peptide show at least equal specificity for EGLN1 and/or EGLN2, a peptide as illustrated in Figure 3 may be selected. For example, as can be seen in Table 1 and Figure 3, the peptide DTDLDLEARApYIPADDDFQ (SEQ ID NO:15) shows higher specificity for EGLN1 and EGLN2 than for EGLN3.

Still further, the present application discloses peptides optimized for EGLN3. For use in an assay wherein the enzyme is EGLN3, or wherein it is desired that the peptide show at least equal specificity for EGLN3, a peptide as illustrated in Figure 4 may be selected. For example, as can be seen in Table 1 and Figure 4, the peptides DTDLDMEALApYIPADDDFQ (SEQ ID NO: 13), DTDLDFEALApYIPADDDFQ (SEQ ID NO:19), and DTDLDIEALApYIPADDDFQ (SEQ ID NO:24) show higher specificity for EGLN3 than for EGLN1 and EGLN2.

Additionally, the present application discloses peptides wherein activity by EGLN1, EGLN2, and EGLN3 are essentially equivalent. For use in an assay wherein direct comparison of EGLN1, EGLN2, and EGLN3 is desired, or wherein it is desired that the peptide show substantially equal specificity for EGLN1, EGLN2, and EGLN3, a peptide as illustrated in Table 1 may be selected. For example, as can be seen in Table 1, the peptide DTDLDLEAFApYIPADDDFQ (SEQ ID NO:14) shows similar specificity for EGLN1,EGLN2, and EGLN3.

The present invention may involve the use of peptides containing amino acid analogs as shown in Table 2. These analogs are used in place of proline, specifically the proline residue modified by hydroxylation in the standard reaction (I). The present invention involves the use of a peptide compnsing the sequence X₁-X₂-X₃-X₄-X₅-Z-X₆, wherein X₁, X₂, and X₃ are independently selected from any amino acid;

X₄ is selected from isoleucine, valine, arginine, phenylalanine, tyrosine, methionine, threonine, lysine, tryptophan, cysteine, asparagine, histidine, serine, alanine, glycine, glutamate, glutamine, or leucine; X₅ is selected from threonine, serine, lysine, glutamine, methionine, isoleucine, arginine, histidine, glutamate, phenylalanine, cysteine, leucine, or alanine; X₆ is selected from phenylalanine or tyrosine; and Z is a proline analog that is not susceptible to hydroxylation by HIF prolyl hydroxyase, for example an analog selected from azetidine-2-carboxylate, 3,4-dehydroproline, b-thioproline, trans-4-hydroxyproline, or azetidine-3-carboxylic acid. Although peptides of any length may be used, the peptide is preferably at least about 10 nucleotides in length, particularly at least about 15 nucleotides in length, and more particularly at least about 20 nucleotides in length. The present invention demonstrates that these analogs, which are not susceptible to hydroxylation by HIF-PH, continue to enable turnover of co-substrate 2-oxoglutarate to succinate and carbon dioxide.

**Table 2**

| **Name** | **Structure** | **Code** |
|---|---|---|
| Azetidine-2-carboxylic acid | 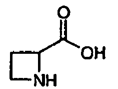 | 8 |
| 3,4-Dehydroproline | 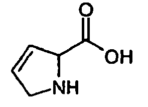 | 4 |
| b-Thioproline | 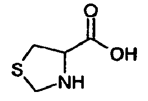 | 5 |
| trans-4-Hydroxyproline | 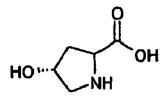 | 2 |
| Azetidine-3-carboxylic acid | 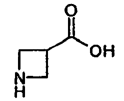 | 3 |
| D-proline | 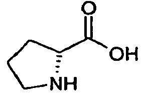 | d |

Further, the various EGLN enzymes show different affinity for the peptides containing proline analogs. The present invention thus provides, in certain embodiments, peptides containing proline analogs optimized for EGLN1 and EGLN2 as shown in Table 3.

**Table 3**

| **SEQ ID NO** | **Sequence** | **Activity (%)*** | |
|---|---|---|---|
| | | **EGLN1** | **EGLN2** |
| 7 | DTDLDLEMLApYIPMDDDFQ | 100.00 | 100.00 |
| 62 | DTDLDLEALA4YIPADDDFQ | 123.94 | 173.02 |
| 63 | DTDLDLEALA8YIPADDDFQ | 78.86 | 62.04 |
| 64 | DTDLDVEAYABYIPADDDFQ | 32.29 | 27.22 |
| 65 | DTDLDTEAYA8YIPADDDFQ | 25.12 | 16.96 |
| 66 | DTDLDVEAFA8YIPADDDFQ | 21.185 | 13.92 |
| 67 | DTDLDIEAIA8YIPADDDFQ | 18.265 | 10.02 |
| 68 | DTDLDTEAFA8YIPADDDFQ | 17.375 | 8.90 |
| 69 | DTDLDTEALA8YIPADDDFQ | 11.39 | 8.52 |
| 70 | DTDLDAEAAA8YIPADDDFQ | 10.32 | 4.16 |
| 71 | DTDLDAEAVA8YIPADDDPQ | 10.03 | 3.36 |
| 72 | DTDLDLEALA5YIPADDDFQ | 8.925 | 7.90 |
| 73 | DTDLDVEAIA8YIPADDDFQ | 7.955 | 2.81 |
| 74 | DTDLDAEATA8YIPADDDFQ | 7.6 | 1.55 |
| 75 | DTDLDAEAIA8YIPADDDFQ | 6.68 | 1.88 |
| 76 | DTDLDIEAVA8YIPADDDPQ | 6.35 | 1.96 |

The peptides shown in Table 3, which retain measurable activity in the present assay, are specifically contemplated for use in assays claimed herein. These peptides are used in a reaction additionally requiring co-substrate 2-oxoglutarate and a reducing agent, e.g., ascorbate; an EGLN enzyme, and oxygen, as described below. The reaction may also include Fe²⁺. The reaction proceeds according to the modified reaction (II), in which 2-oxoglutarate is converted to succinate and carbon dioxide, and the reducing agent is subsequently oxidized, e.g., ascorbate is oxidized to dehydroascorbate. Although the reaction requires the presence of the peptide, the peptide is not modified during the reaction and acts as an enzyme cofactor.

Peptides for use in an assay wherein the enzyme is EGLN1 or EGLN2, or wherein it is desired that the peptide show specific affinity for EGLN1 and/or EGLN2, a peptide as illustrated in Table 3 may be selected. (Also, see Figure 3.) For example, as can be seen in Table 3 and Figure 3, the peptide DTDLDLEALA4YIPADDDFQ (SEQ ID NO:62), wherein the hydroxylated proline is replace by the proline analog 3,4-dehydroproline, shows higher specificity for EGLN1 and EGLN2 than for EGLN3.

The present invention further provides, in certain embodiments, peptides containing proline analogs optimized for EGLN3 as shown in Table 4.

**Table 4**

| **SEQ ID NO** | **Sequence** | **Activity (%)** |
|---|---|---|
| | | **EGLN3** |
| 7 | DTDLDLEMLApYIPMDDDFQ | 119.42 |
| 63 | DTDLDLEALA8YIPADDDFQ | 168.84 |
| 69 | DTDLDTEA8YIPADDDFQ | 80.07 |
| 62 | DTDLDLEALA4YIPADDDFQ | 74.44 |
| 64 | DTDLDVEAYA8YIPADDDFQ | 33.45 |
| 67 | DTDLDIEAIA8YIPADDDFQ | 31.22 |
| 65 | DTDLDTEAYA8YIPADDDFQ | 29.69 |
| 66 | DTDLDVEAFA8YIPADDDFQ | 24.46 |
| 77 | DTDLDVEAVA8YIPADDDFQ | 20.61 |
| 76 | DTDLDIEAVA8YIPADDDFQ | 18.84 |
| 68 | DTDLDTEAPA8YIPADDDFQ | 18.67 |
| 71 | DTDLDAEAVA8YIPADDDFQ | 17.16 |
| 73 | DTDLDVEAIA8YIPADDDFQ | 14.76 |
| 72 | DTDLDLEALA5YIPADDDFQ | 11.07 |
| 78 | DTDLDTEAVA8YIPADDDFQ | 10.31 |
| 75 | DTDLDAEAIA8YIPADDDFQ | 9.72 |

The peptides shown in Table 4, which retain measurable activity in the present assay, are specifically contemplated for use in assays claimed herein. These peptides are used in a reaction additionally requiring 2-oxoglutarate and a reducing agent, e.g., ascorbate; an EGLN enzyme and oxygen, as described below. The reaction may also include Fe²⁺. The reaction proceeds according to the modified reaction (II), in which 2-oxoglutarate is converted to succinate and carbon dioxide, and the reducing agent is subsequently oxidized, e.g., ascorbate is oxidized to dehydroascorbate. Although the reaction requires the presence of the peptide substrate, the peptide is not modified during the reaction and acts as an enzyme cofactor.

For use in an assay wherein the enzyme is EGLN3, or wherein it is desired that the peptide show greater specificity for EGLN3, a peptide as illustrated in Table 4 may be selected. (Also, see Figure 4.) For example, as can be seen in Table 4 and Figure 4, the peptide DTDLDLEALA8YIPADDDFQ (SEQ ID NO:63), wherein the hydroxylated proline is replaced by the proline analog L-azetidine-2-carboxylate, shows higher specificity for EGLN3 than for EGLN1 and EGLN2.

Thus, peptides for use in the following assays include any of the foregoing peptide substrates, particularly those identified in Tables 1 to 4. Peptides contemplated for use in the present assays also include various combinations of the substitutions exemplified in the peptides identified in Tables 1 to 4. For example, combinations of substitutions as shown in Figure 5 demonstrate a range of possible peptides having specificity for EGLNs for use in the modified reaction (II). The panel at the top of Figure 5 demonstrates various modifications that may be made to retain or enhance specificity toward EGLN1 and/or EGLN2, whereas the panel at the bottom of Figure 5 demonstrates various modifications that may be made to retain or enhance specificity toward EGLN3. One skilled in the art can immediately envision, from the information provided above and in Figure 5, a broad range of novel peptides not specifically disclosed herein. For example, peptides DTDLDYEMIApYIPADDDFQ (SEQ ID NO:89), DTDLDWEMVSpYIPADDDFQ (SEQ ID NO:90), DTDLDWEAVSpYIPADDDFQ (SEQ ID NO:91), and DTDLDWEMVApYIPADDDFQ (SEQ ID NO:92) are readily hydroxylated by both EGLN1 and EGLN3.

Use of a peptide in the standard reaction (I) or the modified reaction of the invention (II) is determined primarily by the absence or presence, respectively, of a proline analog in place of the proline residue hydroxylated by the HIF-PH.

Any of the aforementioned peptides may be synthesized chemically utilizing, e.g., FMOC chemistry performed using, e.g., an automated 432A peptide synthesizer (Applied Biosystems), or other technologies known to those skilled in the art.

### ASSAYS

The present invention relates to methods to measure activity of an EGLN enzyme. In one aspect, the methods are assays that comprise combining an EGLN enzyme, 2-oxoacid substrate, and peptide substrate, as provided above under conditions suitable for EGLN enzyme activity; and measuring activity of the enzyme. In certain methods described herein, enzyme activity is measured in the Standard Reaction (I), which converts 2-oxoglutarate to succinate and carbon dioxide, and concomitantly converts unhydroxylated peptide to hydroxylated peptide. The standard assay comprises reaction (I): 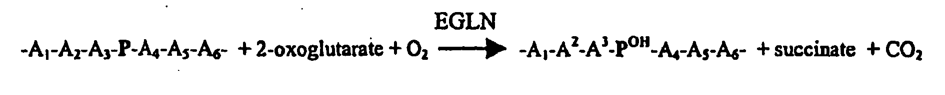 wherein -A₁-A₂-A₃-P-A₄-A₅-A₆- represents the substrate peptide and -A₁-A²-A³-P^{OH}-A₄-A₅-A₆ represents the hydroxylated peptide. The unhydroxylated proline residue is represented as P and the hydroxylated proline is represented as P^{OH}. The particular proline for use in the substrate is preferably the naturally occurring L-proline (p), although the D-proline isomer (d) may be used in some instances. Substrates specifically for use in the present assay include those provided in Table 1. supra, and combinations of these substrates clearly envisioned herein, e.g., SEQ ID NOs 11 and 84 to 86. The co-substrates are combined with enzyme under conditions suitable to support enzyme activity, and activity of the enzyme is then measured. The assay may be used in the absence or presence of additional components, e.g., an agent suspected of potentially increasing or decreasing enzyme activity may be added to the reaction.

In certain embodiments of the methods claimed herein, enzyme activity is measured in the Modified Reaction (II), which converts 2-oxoglutarate to succinate and carbon dioxide, and concomitantly converts a reducing agent (RA), e.g., ascorbate, to its oxidized form, e.g., dehydroascorbate. The modified assay comprises reaction (II): 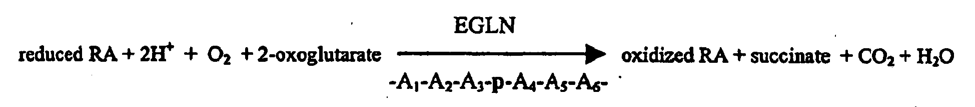 wherein -A₁-A₂-A₃-p-A₄-A₅-A₆- represents the peptide; the proline analog is represented as p. In this reaction, the peptide acts as an enzyme cofactor rather an actual substrate. Preferred peptide analogs include, but are not limited to, both L- and D-isomers, preferably L-isomers, of azedidine-2-carboxylate, 3,4-dehydroproline, and b-thioproline. Additionally, the proline analogs for use in the substrate may include, e.g., both L- and D-isomers, preferably L-isomers, of 2,3-dehydroproline. Peptides specifically for use in the present assay include those provided in Tables 3 and 4, *supra,* and combinations of these substrates clearly envisioned herein. (See, e.g., Figure 5.) In various embodiments, the co-substrates and cofactors are combined with enzyme under conditions suitable to support enzyme activity, and activity of the enzyme is then measured. The assay may be used in the absence or presence of additional components, e.g., an agent suspected of potentially increasing or decreasing enzyme activity may be added to the reaction; such use of the modified assay is clearly encompassed by the present invention.

The assay may also include iron. In general, ferrous ions are provided in a range of from about 0 to 500 µM, e.g., from 25 to 250 µM, and in particular from 50 to 200 µM. Ferrous ions may be supplied in any suitable form including, e.g., ferrous chloride, ferrous sulfate, etc. The 2-oxoglutarate is provided in a range of from about 0.1 to 100 µM, e.g., from 10 to 100 µM; and may be supplied in any suitable form including, e.g., a salt. Generally, and specifically in standard reaction (I), ascorbate is provided in a range of from about 0.1 to 10 mM, e.g., from 0.5 to 5 mM; and may be supplied in any suitable form including, e.g., a salt, e.g., sodium ascorbate. The ascorbate may be replaced by another reducing agent, e.g., potassium ferrocyanide, in standard reaction (I) or, more preferably, in modified reaction (II).

Enzyme activity can be measured by monitoring the consumption of one or more reaction substrates, or accumulation of one or more reaction products. For example, enzyme activity in either the standard reaction (I) or the modified reaction (II) can be measured using a modified version of a method described by Kivirikko and Myllyla (1982, Methods Enzymol 82:245-304), which is based on the decarboxylation of 2-oxo[1-¹⁴C]glutaric acid. In this method, radioactive ¹⁴CO₂ produced during the reaction is captured on base-impregnated filter papers and the captured radioactivity determined by liquid scintillation counting. Enzyme activity in standard reaction (I) may be measured as described in (Reference) Example 2 (*infra*).

In various embodiments, enzyme activity within the modified reaction (II) may be measured by disappearance of the reduced form of the reducing agent or appearance of the oxidized form of the agent. Any suitable reducing agent may be used in modified reaction (II); reducing agents specifically contemplated for use by the present invention may be selected from ascorbate and ferrocyanide. For example, when ascorbate is used in modified reaction (II), disappearance of ascorbate and/or appearance of dehydroascorbate may be measured, e.g., using HPLC. In particular embodiments, ascorbate may be added to modified reaction (II) in limiting quantity, and ascorbate is regenerated during the reaction with concomitant oxidation of NADH to NAD⁺ using a modification of the method described by Diliberto and Allen (1981, J Biol Chem 256:3385-3393). The reaction additionally includes, e.g., semidehydroascorbate reductase, which catalyzes the NADH-dependent reduction of semidehyro-ascorbate to ascorbate. HIF-PH activity is measured indirectly by consumption of NADH, which produces a reduction in absorbance at a wavelength of 340 nm. When modified reaction (II) is carried out using potassium ferrocyanide, conversion of the reducing agent to the oxidized form, potassium ferricyanide, can be measured directly using spectrophotometry. In a particular embodiment, enzyme activity in modified reaction (II) was measured as described in Example 3 (*infra*).

### TEST AGENTS

Agents for use in the method to identity modulators of EGLN enzyme activity include, but are not limited to, natural or synthetic chemical compounds. Such compounds or agents may be obtained as extracts, which contain several characterized or uncharacterized components, obtained from plants, microbes, or other organisms; or they may be complex libraries of polypeptides or small molecules, such as molecules from commercially available combinatorial libraries, or the like. Compounds or agents may also be obtained as a focused chemical library of candidate molecules; for example, focused libraries specific for kinase inhibitors have been described. (See, e.g., Gray et al. (1998) Science 281:533-538; and Chang et al. (1999) Chem Biol 6:361-375.)

These and other embodiments of the present invention will readily occur to those of ordinary skill in the art in view of the disclosure herein.

### EXAMPLES

The invention will be further understood by reference to the following examples, which are intended to be purely exemplary of the invention. These examples are provided solely to illustrate the claimed invention. The present invention is not limited in scope by the exemplified embodiments, which are intended as illustrations of single aspects of the invention only. Various modifications of the invention in addition to those described herein will become apparent to those skilled in the art from the foregoing description and accompanying figures.

### (Synthesis) Example 1: Peptides

Peptides used in the present invention were synthesized by Mimotopes (San Diego CA) and SynPep Corporation (Dublin CA). Activity was measured relative to a control peptide, DTD20:
DTDLDLEMLAPYIPMDDDFQ (DTD20)

DTD20 is most similar to the HIF-1α C-ODD domain, however extensive homology exists between all HIFα family members within this region. In some assays provided herein, activity was measured in the presence of peptide DLD19:
DLDLEALAPYIPADDDFQL (DLD19)

In DLD19, methionine (M) residues were changed to alanine (A) residues due to potential oxidation of methionine during the assay reaction. Peptides used in the assay may additionally contain N-terminal and/or C-terminal blocking groups, e.g., N-terminal acetyl groups, C-terminal amide groups, etc. Enzyme activity in the presence of substrate peptides is provided *supra* as percent enzyme activity relative to enzyme activity in the presence of control peptide DTD20.

### (Reference) Example 2: Standard reaction (I)

In all experiments, percent enzyme activity, e.g., as measured by substrate turnover, was less than 30% to insure all data obtained were in the linear range. Enzyme activity in standard reaction (I) was measured in a reaction volume of 1 mL containing EGLN enzyme preparation (10-400 µL), 0.05 µmole hydroxylatable peptide substrate, 0.1 µmole of 2-oxo[1-¹⁴C]glutaric acid (160,000 dpm), 1 µmole of ascorbic acid, 60 µg of catalyse and 50 µmole of HEPES buffer adjusted to pH 7.4 at 25° C. Optionally, 0.04 µmole FeSO₄ was also be added to the reaction mix. Reactions were carried out at 37° C for 20 minutes. In various reactions, ascorbate was replaced by 0.1 to 15 mM potassium ferrocyanide. Results are provided in Table 1 *(supra)* and Figures 3 and 4.

### Example 3: Modified reaction (II)

Enzyme activity in modified reaction (II) was measured as described in (Reference) Example 2 (*supra*), except non-hydroxylatable peptide was used, and reducing agent was added to the reaction. In various modified reaction (II), 0.1 to 5.0 mM potassium ferrocyanide was used as the reducing agent, and following incubation of the reaction mix, a 105 µl aliquot of the reaction solution was transferred to a clear plate and optical density was measured at a wavelength of 405 nm. (See Figure 6.) When ascorbate is used as the reducing agent, disappearance of ascorbate and/or appearance of dehydroascorbate is measured using HPLC.

Various modifications of the invention, in addition to those shown and described herein, will become apparent to those skilled in the art from the foregoing description.

## Claims

1. A method of measuring the activity of an EGLN enzyme, the method comprising
(a) combining an EGLN enzyme with 2-oxoglutarate, a reducing agent and a peptide under conditions suitable for EGLN enzyme activity; and
(b) measuring activity of the enzyme,
wherein the peptide comprises the sequence X₁-X₂-X₃-X₄-X₅-Z-X₆, wherein
X₁, X₂, and X₃ are independently selected from any amino acid;
X₄ is selected from isoleucine, valine, arginine, phenylalanine, tyrosine, methionine, threonine, lysine, tryptophan, cysteine, asparagine, histidine, serine, alanine, glycine, glutamate, glutamine, or leucine;
X₅ is selected from threonine, serine, lysine, glutamine, methionine, isoleucine, arginine, histidine, glutamate, phenylalanine, cysteine, leucine, or alanine;
X₆ is selected from phenylalanine or tyrosine; and
Z is a proline analog that is not susceptible to hydroxylation by HIF prolyl hydroxylase.

2. A method of identifying an agent that modulates activity of an EGLN enzyme, the method comprising
(a) combining an EGLN enzyme with a test agent, 2-oxoglutarate, a reducing agent and a peptide under conditions suitable, in the absence of agent, for EGLN enzyme activity,
wherein the peptide comprises the sequence X₁-X₂-X₃-X₄-X₅-Z-X₆, wherein
X₁, X₂, and X₃ are independently selected from any amino acid;
X₄ is selected from isoleucine, valine, arginine, phenylalanine, tyrosine, methionine, threonine, lysine, tryptophan, cysteine, asparagine, histidine, serine, alanine, glycine, glutamate, glutamine, or leucine;
X₅ is selected from threonine, serine, lysine, glutamine, methionine, isoleucine, arginine, histidine, glutamate, phenylalanine, cysteine, leucine, or alanine,
X₆ is selected from phenylalanine or tyrosine, and
Z is a proline analog that is not susceptible to hydroxylation by HIF prolyl hydroxylase; and
(b) measuring activity of the enzyme; and
(c) comparing the activity of the enzyme in the presence of the agent to the activity of the enzyme in the absence of the agent, wherein a change in the activity of the enzyme in the presence of the agent relative to the activity of the enzyme in the absence of the agent is indicative of an agent that modulates EGLN activity.

3. The method of claim 1 or 2, wherein the reducing agent is selected from the group consisting of ascorbate and potassium ferrocyanide.

4. The method of any one of claims 1 to 3, wherein the combining step additionally comprises iron.

5. The method of any of claims 1 to 4, wherein the EGLN enzyme comprises the sequence of SEQ ID NO: 3.

6. The method of claim 5, wherein the EGLN enzyme comprises the sequence of SEQ ID NO: 2.

7. The method of claim 6, wherein the EGLN enzyme is selected from the group consisting of EGLN1, EGLN2, EGLN3, and active fragments of EGLN1, EGLN2, and EGLN3.

8. The method of any of claims 1 to 7, wherein the peptide is selected from the group consisting of SEQ ID NOs: 62-78.

9. The method of any of claims 1 to 4, wherein the EGLN enzyme is selected from the group comprising EGLN1, an active fragment of EGLN1, EGLN2, and an active fragment of EGLN2; and the peptide comprises the sequence X₁-X₂-X₃-X₄-X₅-Z-X₆, wherein
X₁ is selected from tyrosine, tryptophan, methionine, isoleucine, phenylalanine, aspartate, alanine, glutamate, cysteine, proline, glycine, and leucine;
X₂, and X₃ are independently selected from any amino acid;
X₄ is selected from isoleucine, valine, arginine, phenylalanine, tyrosine, methionine, threonine, lysine, tryptophan, cysteine, asparagine, histidine, serine, alanine, glycine, glutamate, glutamine, or leucine;
X₅ is selected from threonine, serine, lysine, glutamine, methionine, or alanine;
X₆ is selected from phenylalanine or tyrosine; and
Z is a proline analog that is not susceptible to hydroxylation by HIF prolyl hydroxylase.

10. The method of claim 9, wherein the peptide is selected from the group consisting of SEQ ID NOs: 62 and 63.

11. The method of any of claims 1 to 4, wherein the EGLN enzyme is EGLN3 or an active fragment of EGLN3.

12. The method of claim 11, wherein the peptide is selected from the group consisting of SEQ ID NOs: 62, 63, 69 and 72.

13. The method of any of claims 1 to 7, wherein Z is azetidine-2-carboxylate.

14. The method of claim 13, wherein the peptide is selected from the group consisting of SEQ ID NOs: 63, 64, 65, 66, 67, 68, 69,70, 71, 73, 74, 75, 76, 77, and 78.

15. The method of any of claims 1 to 7, wherein Z is 3,4-dehydroproline.

16. The method of claim 15, wherein the peptide is SEQID NO: 62.

17. The method of any of claims 1 to 7, wherein Z is b-thioproline.

18. The method of claim 17, wherein the peptide is SEQ ID NO: 72.

19. The method of any of claims 1 to 18, wherein measuring EGLN activity comprises measuring carbon dioxide produced by the reaction, wherein the amount of carbon dioxide produced is directly proportional to the activity of the EGLN enzyme.

20. The method of any of claims 1 to 7 and 13 to 18, wherein measuring EGLN activity comprises measuring conversion of the reducing agent to its oxidized form during the reaction, wherein the amount of reducing agent that is oxidized is directly proportional to the activity of the EGLN enzyme.

21. The method of any of claims 1 to 20, wherein the peptide is at least about 20 amino acids in length.

22. The method of any of the preceding claims, wherein Z is selected from L- and D-isomers of azetidine-2-carboxylate, 3,4-dehydroproline, b-thioproline and 2,3-dehydroproline.

23. The method of claim 22, wherein Z is an L-isomer.

24. The method of any of claims 1-21, wherein Z is selected from *trans*-4-hydroxyproline, azetidine-3-carboxylic acid and D-proline.

## Patentansprüche

1. Verfahren zum Messen der Aktivität eines EGLN-Enzyms, das Verfahren umfassend
(a) das Vereinigen eines EGLN-Enzyms mit 2-Oxoglutarat, einem Reduktionsmittel und einem Peptid unter Bedingungen, die für EGLN-Enzymaktivität geeignet sind, und
(b) das Messen der Aktivität des Enzyms,
wobei das Peptid die Sequenz X₁-X₂-X₃-X₄-X₅-Z-X₆ umfaßt, wobei
X₁, X₂ und X₃ unabhängig ausgewählt sind aus einer beliebigen Aminosäure,
X₄ ausgewählt ist aus Isoleucin, Valin, Arginin, Phenylalanin, Tyrosin, Methionin, Threonin, Lysin, Tryptophan, Cystein, Asparagin, Histidin, Serin, Alanin, Glycin, Glutamat, Glutamin oder Leucin,
X₅ ausgewählt ist aus Threonin, Serin, Lysin, Glutamin, Methionin, Isoleucin, Arginin, Histidin, Glutamat, Phenylalanin, Cystein, Leucin oder Alanin,
X₆ ausgewählt ist aus Phenylalanin oder Tyrosin, und
Z ein Prolin-Analogon ist, das nicht für Hydroxylierung durch HIF-Prolylhydroxylase empfänglich ist.

2. Verfahren zum Identifizieren eines Mittels, das die Aktivität eines EGLN-Enzyms moduliert, das Verfahren umfassend
(a) das Vereinigen eines EGLN-Enzyms mit einem Testmittel, 2-Oxoglutarat, einem Reduktionsmittel und einem Peptid unter Bedingungen, die in Abwesenheit des Mittels für EGLN-Enzymaktivität geeignet sind,
wobei das Peptid die Sequenz X₁-X₂-X₃-X₄-X₅-Z-X₆ umfaßt, wobei
X₁, X₂ und X₃ unabhängig ausgewählt sind aus einer beliebigen Aminosäure,
X₄ ausgewählt ist aus Isoleucin, Valin, Arginin, Phenylalanin, Tyrosin, Methionin, Threonin, Lysin, Tryptophan, Cystein, Asparagin, Histidin, Serin, Alanin, Glycin, Glutamat, Glutamin oder Leucin,
X₅ ausgewählt ist aus Threonin, Serin, Lysin, Glutamin, Methionin, Isoleucin, Arginin, Histidin, Glutamat, Phenylalanin, Cystein, Leucin oder Alanin,
X₆ ausgewählt ist aus Phenylalanin oder Tyrosin, und
Z ein Prolin-Analogon ist, das nicht für Hydroxylierung durch HIF-Prolylhydroxylase empfänglich ist, und
(b) Messen der Aktivität des Enzyms, und
(c) Vergleichen der Aktivität des Enzyms in Anwesenheit des Mittels mit der Aktivität des Enzyms in Abwesenheit des Mittels, wobei eine Veränderung in der Aktivität des Enzyms in Anwesenheit des Mittels relativ zur Aktivität des Enzyms in Abwesenheit des Mittels ein Mittel anzeigt, das die EGLN-Aktivität moduliert.

3. Verfahren nach Anspruch 1 oder 2, wobei das Reduktionsmittel ausgewählt ist aus der Gruppe, bestehend aus Ascorbinsäure und Kaliumferrocyanid.

4. Verfahren nach einem der Ansprüche 1 bis 3, wobei der Vereinigungsschritt zusätzlich Eisen umfaßt.

5. Verfahren nach einem der Ansprüche 1 bis 4, wobei das EGLN-Enzym die Sequenz von SEQ ID NR: 3 umfaßt.

6. Verfahren nach Anspruch 5, wobei das EGLN-Enzym die Sequenz von SEQ ID NR: 2 umfaßt.

7. Verfahren nach Anspruch 6, wobei das EGLN-Enzym ausgewählt ist aus der Gruppe, bestehend aus EGLN1, EGLN2, EGLN3 und aktiven Fragmenten von EGLN1, EGLN2 und EGLN3.

8. Verfahren nach einem der Ansprüche 1 bis 7, wobei das Peptid ausgewählt ist aus der Gruppe, bestehend aus SEQ ID NRn: 62 bis 78.

9. Verfahren nach einem der Ansprüche 1 bis 4, wobei das EGLN-Enzym ausgewählt ist aus der Gruppe, umfassend EGLN1, ein aktives Fragment von EGLN1, EGLN2 und einem aktiven Fragment von EGLN2, und das Peptid die Sequenz X₁-X₂-X₃-X₄-X₅-Z-X₆ umfaßt, wobei
X₁ ausgewählt ist aus Tyrosin, Tryptophan, Methionin, Isoleucin, Phenylalanin, Aspartat, Alanin, Glutamat, Cystein, Prolin, Glycin und Leucin,
X₂ und X₃ unabhängig ausgewählt sind aus einer beliebigen Aminosäure,
X₄ ausgewählt ist aus Isoleucin, Valin, Arginin, Phenylalanin, Tyrosin, Methionin, Threonin, Lysin, Tryptophan, Cystein, Asparagin, Histidin, Serin, Alanin, Glycin, Glutamat, Glutamin oder Leucin,
X₅ ausgewählt ist aus Threonin, Serin, Lysin, Glutamin, Methionin oder Alanin,
X₆ ausgewählt ist aus Phenylalanin oder Tyrosin, und
Z ein Prolin-Analogon ist, das nicht für Hydroxylierung durch HIF-Prolyl-Hydroxylase empfänglich ist.

10. Verfahren nach Anspruch 9, wobei das Peptid ausgewählt ist aus der Gruppe, bestehend aus SEQ ID NRn: 62 und 63.

11. Verfahren nach einem der Ansprüche 1 bis 4, wobei das EGLN-Enzym EGLN3 oder ein aktives Fragment von EGLN3 ist.

12. Verfahren nach Anspruch 11, wobei das Peptid ausgewählt ist aus der Gruppe, bestehend aus SEQ ID NRn: 62, 63, 69 und 72.

13. Verfahren nach einem der Ansprüche 1 bis 7, wobei Z Azetidin-2-carboxylat ist.

14. Verfahren nach Anspruch 13, wobei das Peptid ausgewählt ist aus der Gruppe, bestehend aus SEQ ID NRn: 63, 64, 65, 66, 67, 68, 69, 70, 71, 73, 74, 75, 76, 77 und 78.

15. Verfahren nach einem der Ansprüche 1 bis 7, wobei Z 3,4-Dehydroprolin ist.

16. Verfahren nach Anspruch 15, wobei das Peptid SEQ ID NR: 62 ist.

17. Verfahren nach einem der Ansprüche 1 bis 7, wobei Z b-Thioprolin ist.

18. Verfahren nach Anspruch 17, wobei das Peptid SEQ ID NR: 72 ist.

19. Verfahren nach einem der Ansprüche 1 bis 18, wobei das Messen der EGLN-Aktivität das Messen von Kohlendioxid, das durch die Reaktion hergestellt wird, umfaßt, wobei die Menge von Kohlendioxid, die hergestellt wird, direkt proportional zu der Aktivität des EGLN-Enzyms ist.

20. Verfahren nach einem der Ansprüche 1 bis 7 und 13 bis 18, wobei das Messen der EGLN-Aktivität, das Messen der Umwandlung des Reduktionsmittels in seine oxidierte Form während der Reaktion umfaßt, wobei die Menge von Reduktionsmittel, das oxidiert wird, direkt proportional zu der Aktivität des EGLN Enzyms ist.

21. Verfahren nach einem der Ansprüche 1 bis 20, wobei das Peptid mindestens etwa 20 Aminosäuren lang ist.

22. Verfahren nach einem der vorstehenden Ansprüche, wobei Z ausgewählt ist aus L- und D-Isomeren von Azetidin-2-carboxylat, 3,4-Dehydroprolin, b-Thioprolin und 2,3-Dehydroprolin.

23. Verfahren nach Anspruch 22, wobei Z ein L-Isomer ist.

24. Verfahren nach einem der Ansprüche 1 bis 21, wobei Z ausgewählt ist aus *trans*-4-Hydroxyprolin, Azetidin-3-carbonsäure und D-Prolin.

## Revendications

1. Procédé de mesure de l'activité d'une enzyme EGLN, le procédé comprenant les étapes de
(a) combinaison d'une enzyme EGLN avec un 2-oxoglutarate, un agent de réduction et un peptide dans des conditions convenables pour l'activité de l'enzyme EGLN ; et
(b) mesures de l'activité de l'enzyme,
dans lequel le peptide comprend la séquence X₁-X₂-X₃-X₄-X₅-Z-X₆, où
X₁, X₂, et X₃ sont indépendamment choisis parmi un acide aminé quelconque ;
X₄ est choisi parmi isoleucine, valine, arginine, phénylalanine, tyrosine, méthionine, thréonine, lysine, tryptophane, cystéine, asparagine, histidine, sérine, alanine, glycine, glutamate, glutamine, ou leucine ;
X₅ est choisi parmi thréonine, sérine, lysine, glutamine, méthionine, isoleucine, arginine, histidine, glutamate, phénylalanine, cystéine, leucine, ou alanine ;
X₆ est choisi parmi phénylalanine ou tyrosine ; et
Z est un analogue de proline qui n'est pas sensible à l'hydroxylation par la prolyl hydroxylase de HIF.

2. Procédé d'identification d'un agent qui module l'activité d'une enzyme EGLN, le procédé comprenant les étapes de
(a) combinaison d'une enzyme EGLN avec un agent d'essai, un 2-oxoglutarate, un agent réducteur et un peptide dans des conditions convenables, en l'absence d'agent, pour l'activité de l'enzyme EGLN,
dans lequel le peptide comprend la séquence X₁-X₂-X₃-X₄-X₅-Z-X₆, où
X₁, X₂, et X₃ sont indépendamment choisis parmi un acide aminé quelconque ;
X₄ est choisi parmi isoleucine, valine, arginine, phénylalanine, tyrosine, méthionine, thréonine, lysine, tryptophane, cystéine, asparagine, histidine, sérine, alanine, glycine, glutamate, glutamine, ou leucine ;
X₅ est choisi parmi thréonine, sérine, lysine, glutamine, méthionine, isoleucine, arginine, histidine, glutamate, phénylalanine, cystéine, leucine, ou alanine,
X₆ est choisi parmi phénylalanine ou tyrosine, et
Z est un analogue de proline qui n'est pas sensible à l'hydroxylation par la prolyl hydroxylase de HIF ; et
(b) mesure de l'activité de l'enzyme ; et
(c) comparaison de l'activité de l'enzyme en présence de l'agent à l'activité de l'enzyme en l'absence de l'agent, dans lequel une variation d'activité de l'enzyme en présence de l'agent par rapport à l'activité de l'enzyme en l'absence de l'agent est indicatrice d'un agent qui module l'activité EGLN.

3. Procédé selon les revendications 1 ou 2, dans lequel l'agent réducteur est choisi dans le groupe constitué par l'ascorbate et le ferrocyanure de potassium.

4. Procédé selon l'une quelconque des revendications 1 à 3, dans lequel l'étape de combinaison comprend, en plus, du fer.

5. Procédé selon l'une quelconque des revendications 1 à 4, dans lequel l'enzyme EGLN comprend la séquence SÉQ ID No.: 3.

6. Procédé selon la revendication 5, dans lequel l'enzyme EGLN comprend la séquence SÉQ ID No.: 2.

7. Procédé selon la revendication 6, dans lequel l'enzyme EGLN est choisie dans le groupe constitué par EGLN1, EGLN2, EGLN3, et des fragments actifs d'EGLN1, EGLN2, et EGLN3.

8. Procédé selon l'une quelconque des revendications 1 à 7, dans lequel le peptide est choisi dans le groupe constitué par SÉQ ID No.: 62-78.

9. Procédé selon l'une quelconque des revendications 1 à 4, dans lequel l'enzyme EGLN est choisie dans le groupe comprenant EGLN1, un fragment actif d'EGLN1, EGLN2, et un fragment actif d'EGLN2 ; et le peptide comprend la séquence X₁-X₂-X₃-X₄-X₅-Z-X₆, où
X₁ est choisi parmi tyrosine, tryptophane, méthionine, isoleucine, phénylalanine, aspartate, alanine, glutamate, cystéine, proline, glycine, et leucine ;
X₂ et X₃ sont indépendamment choisis parmi un acide aminé quelconque ;
X₄ est choisi parmi isoleucine, valine, arginine, phénylalanine, tyrosine, méthionine, thréonine, lysine, tryptophane, cystéine, asparagine, histidine, sérine, alanine, glycine, glutamate, glutamine, ou leucine ;
X₅ est choisi parmi thréonine, sérine, lysine, glutamine, méthionine, ou alanine,
X₆ est choisi parmi phénylalanine ou tyrosine ; et
Z est un analogue de proline qui n'est pas sensible à l'hydroxylation par la prolyl hydroxylase de HIF.

10. Procédé selon la revendication 9, dans lequel le peptide est choisi dans le groupe constitué par SÉQ ID No.: 62 et 63.

11. Procédé selon l'une quelconque des revendications 1 à 4, dans lequel l'enzyme EGLN est EGLN3 ou un fragment actif d'EGLN3.

12. Procédé selon la revendication 11, dans lequel le peptide est choisi dans le groupe constitué par SÉQ ID No.: 62, 63, 69 et 72.

13. Procédé selon l'une quelconque des revendications 1 à 7, dans lequel Z est un 2-carboxylate d'azétidine.

14. Procédé selon la revendication 13, dans lequel le peptide est choisi dans le groupe constitué par SÉQ ID No.: 63, 64, 65, 66, 67, 68, 69, 70, 71, 73, 74, 75, 76, 77 et 78.

15. Procédé selon l'une quelconque des revendications 1 à 7, dans lequel Z est la 3,4-déshydroproline.

16. Procédé selon la revendication 15, dans lequel le peptide est SÉQ ID No.: 62.

17. Procédé selon l'une quelconque des revendications 1 à 7, dans lequel Z est la b-thioproline.

18. Procédé selon la revendication 17, dans lequel le peptide est SÉQ ID No.: 72.

19. Procédé selon l'une quelconque des revendications 1 à 18, dans lequel la mesure de l'activité EGLN comprend la mesure du dioxyde de carbone produit par la réaction, la quantité de dioxyde de carbone produite étant directement proportionnelle à l'activité de l'enzyme EGLN.

20. Procédé selon l'une quelconque des revendications 1 à 7 et 13 à 18, dans lequel la mesure de l'activité EGLN comprend la mesure de la conversion de l'agent de réduction en sa forme oxydée pendant la réaction, la quantité d'agent réducteur qui est oxydée étant directement proportionnelle à l'activité de l'enzyme EGLN.

21. Procédé selon l'une quelconque des revendications 1 à 20, dans lequel le peptide est au moins long d'environ 20 acides aminés.

22. Procédé selon l'une quelconque des revendications précédentes, dans lequel Z est choisi parmi les isomères L et D du 2-carboxylate d'azétidine, de la 3,4-déshydroproline, de la b-thioproline et de la 2,3-déshydroproline.

23. Procédé selon la revendication 22, dans lequel Z est un isomère L.

24. Procédé selon l'une quelconque des revendications 1 à 21, dans lequel Z est choisi parmi la *trans-*4-hydroxyproline, l'acide azétidine-3-carboxylique et la D-proline.
